# EUROPEAN PATENT APPLICATION

(11) **EP 4 445 881 A2**
(43) Date of publication of application: **16.10.2024**
(21) Application number: 24190517.3
(22) Date of filing: 22.12.2021
(51) Int. Cl.: A61F 5/443

(54) **DEVICES AND SYSTEMS FOR URINE COLLECTION**

(30) Priority: 23.12.2020 US 202063129729 P
(62) Divisional of application: 21847861.8
(71) Applicant: Sage Products, LLC, Cary, IL 60013 (US)
(72) Inventor: KHARKAR, Prathamesh M, Cary, 60013 (US); EKLUND, Brian J., Cary, 60013 (US); BLABAS, Brett C., Cary, 60013 (US)
(74) Representative: Schott, Jakob Valentin

(57) **Abstract**

A urine collection system includes a first fluid collection device with (i) a first internal cavity, (ii) a first inlet in fluid communication with the first internal cavity, and (iii) a first outlet in fluid communication with the first internal cavity. The urine collection system further includes a tube in fluid communication with the first outlet. The urine collection system further includes a second fluid collection device with (i) a second internal cavity, (ii) a permeable membrane positioned within the second internal cavity, thereby separating the second internal cavity into a first chamber and a second chamber, (iii) a second inlet in fluid communication with the first internal cavity, and (iv) a second outlet in fluid communication with the second inlet of the second fluid collection device.

## Description

### CROSS-REFERENCE TO RELATED APPLICATION

The present application claims the benefit of priority to U.S. Provisional Application No. 63/129,729, filed December 23, 2020, the contents of which is hereby incorporated by reference in its entirety.

### FIELD

The present disclosure generally relates to devices and systems for collecting urine discharged from the body of a user and carrying the urine away from the body.

### BACKGROUND

Under various circumstances, a user may have limited or impaired mobility such that ordinary urinary functions and processes are rendered difficult (or impossible). For example, a person may have impaired mobility due to a disability or may be bedridden due to an injury or illness. In another example, a person may be subject to restricted occupational conditions under which the person has limited mobility. Also, for example, urine collection may be needed for monitoring purposes, such as for monitoring inputs and outputs in a clinical setting (e.g., in an intensive care unit, or for other clinical and/or laboratory testing).

Various approaches have been developed to address some of the problems or circumstances related to impaired or restricted urinary processes. However, prior approaches suffer from problems or limitations of their own. Internal urinary catheters, for example, can address problems arising from urinary incontinence or limited mobility, but urinary catheters can often be uncomfortable and can contribute to complications (for example, infections). Bed pans, as another example, are containers occasionally used for collecting urinary output of a bedridden person (such as a patient at a health care facility), but bed pans can contribute to patient discomfort, spillage, and issues related to sanitation or hygiene.

Other more recent approaches to urinary collection have been developed, which include a urine collection device configured to be placed external to, but in contact with the body for collecting and directing a fluid receptacle. However, the recent approaches also present challenges, such as in maintaining the placement of the device in appropriate contact with the body of a user, resulting in potential leakage and patient discomfort.

### SUMMARY

In an example, a urine collection device includes a collection member extending from a proximal end to a distal end. The proximal end includes an opening that provides access to an internal cavity of the collection member. The internal cavity comprises a first chamber in fluid communication with a second chamber. The urine collection device also includes a spacer in the second chamber, a first attachment member, a second attachment member, and an outlet. The first attachment member extends from a bottom wall of the collection member at the proximal end. The first attachment member also defines an aperture. The second attachment member extends from a top wall of the collection member at the proximal end. The outlet is suitable for egressing urine from the internal cavity of the collection member. The collection member is suitable to (i) direct urine from the first chamber to the second chamber and (ii) direct the urine in the second chamber distally toward the outlet.

In another example, a urine collection system includes a first fluid collection device with (i) a first internal cavity to collect urine discharged from a body of a user, (ii) a first inlet in fluid communication with the first internal cavity, and (iii) a first outlet in fluid communication with the first internal cavity. The urine collection system further includes a tube in fluid communication with the first outlet. The urine collection system further includes a second fluid collection device having a first end and a second end opposite the first end, the second fluid collection device with (i) a second internal cavity defined between the first end and the second end of the second fluid collection device, (ii) a permeable membrane positioned within the second internal cavity between the first end and the second end of the second fluid collection device, thereby separating the second internal cavity into a first chamber and a second chamber, (iii) a second inlet in fluid communication with the first internal cavity of the first fluid collection device, wherein the second inlet is positioned between the first end of the second fluid collection device and the permeable membrane, and (iv) a second outlet in fluid communication with the second inlet of the second fluid collection device, wherein the second outlet is positioned between the first end of the second fluid collection device and the permeable membrane.

In yet another example, a urine collection device includes an internal cavity to collect urine discharged from a body of a user, an outlet in fluid communication with the internal cavity, and a spacer positioned between the internal cavity and the outlet. The spacer comprises a pliable film including a plurality of protrusions and a plurality of through-holes, and the spacer is in fluid communication with the internal cavity and the outlet such that urine is directed from the internal cavity, through the spacer, and to the outlet.

The features, functions, and advantages that have been discussed can be achieved independently in various embodiments or may be combined in yet other embodiments further details of which can be seen with reference to the following description and drawings.

### BRIEF DESCRIPTION OF THE FIGURES

The novel features believed characteristic of the illustrative embodiments are set forth in the appended claims. The illustrative embodiments, however, as well as a preferred mode of use, further objectives and descriptions thereof, will best be understood by reference to the following detailed description of an illustrative embodiment of the present disclosure when read in conjunction with the accompanying drawings, wherein:
Figure 1A illustrates a perspective view of a urine collection device, according to an example.
Figure 1B illustrates another perspective view of the urine collection device shown in Figure 1A, according to an example.
Figure 1C illustrates a cross-sectional view of the urine collection device shown in Figure 1B, according to an example.
Figure 1D illustrates an exploded view of the urine collection device shown in Figure 1A, according to an example.
Figure 2A illustrates a top view of a urine collection device, according to another example.
Figure 2B illustrates another top view of the urine collection device shown in Figure 2A, according to an example.
Figure 2C illustrates a side view of the urine collection device shown in Figure 2A, according to an example.
Figure 3A illustrates a first stage of a process for using the urine collection device shown in Figures 2A-2C, according to an example.
Figure 3B illustrates a second stage of a process for using the urine collection device shown in Figures 2A-2C, according to an example.
Figure 3C illustrates a third stage of a process for using the urine collection device shown in Figures 2A-2C, according to an example.
Figure 4 illustrates a top view of a urine collection device, according to another example.
Figure 5 illustrates a side view of a port, according to an example.
Figure 6 illustrates a side view of a urine collection device, according to another example.
Figure 7A illustrates a perspective view of a urine collection device, according to another example.
Figure 7B illustrates a top view of the urine collection device shown in Figure 7A, according to an example.
Figure 8A illustrates an exploded view of a urine collection device, according to another example.
Figure 8B illustrates a perspective view of the urine collection device shown in Figure 16A, according to another example.
Figure 8C illustrates another perspective view of the urine collection device shown in Figure 8B, according to an example.
Figure 8D illustrates a cross-sectional view of the urine collection device shown in Figure 8C, according to an example.
Figure 9 illustrates an inner wall of a urine collection device, according to an example.
Figure 10A illustrates a first side of a permeable layer of a urine collection device, according to an example.
Figure 10B illustrates a second side of the permeable layer shown in Figure 18A, according to an example.
Figure 11A illustrates a perspective view of a urine collection device, according to another example.
Figure 11B illustrates a top plan view of the urine collection device shown in Figure 11A, according to an example.
Figure 11C illustrates a bottom plan view of the urine collection device shown in Figure 11A, according to an example.
Figure 11D illustrates a first side elevation view of the urine collection device shown in Figure 11A, according to an example.
Figure 11E illustrates a second side elevation view of the urine collection device shown in Figure 11A, according to an example.
Figure 11F illustrates a third side elevation view of the urine collection device shown in Figure 11A, according to an example.
Figure 11G illustrates a fourth side elevation view of the urine collection device shown in Figure 11A, according to an example.
Figure 12A illustrates a flowchart for a process for collecting urine, according to an example.
Figure 12B illustrates a flowchart for a process for collecting urine that can be performed with the process shown in Figure 12A, according to an example.
Figure 13 illustrates simplified diagram of a system for collecting urine, according to an example.
Figure 14A illustrates another example urine collection device, according to an example.
Figure 14B illustrates an example spacer of the urine collection device of Figure 14A, according to an example.
Figure 15 illustrates a urine collection device, according to another example.
Figure 16 illustrates a urine collection device, according to another example.
Figure 17A illustrates a urine collection device including tabs in a decoupled state, according to another example.
Figure 17B illustrates the urine collection device of Figure 17A with one of the tabs in a coupled state, according to an example.
Figure 18A illustrates a urine collection system in a first mode of operation, according to an example.
Figure 18B illustrates the urine collection system of Figure 18A in a second mode of operation, according to an example.
Figure 18C illustrates another urine collection system, according to an example.
Figure 18D illustrates an example second fluid collection device of the urine collection systems of Figures 18A-18C, according to an example.
Figure 19 illustrates a urine collection device, according to another example.

### DETAILED DESCRIPTION

Disclosed embodiments will now be described more fully hereinafter with reference to the accompanying drawings, in which some, but not all of the disclosed embodiments are shown. Indeed, several different embodiments may be described and should not be construed as limited to the embodiments set forth herein. Rather, these embodiments are described so that this disclosure will be thorough and complete and will fully convey the scope of the disclosure to those skilled in the art.

The urine collection devices disclosed herein divert urine away from the skin of patients to provide urine management with an external suction line and a fluid collection reservoir (e.g., a fluid collection canister). In some implementations, a vacuum pump can operate at a pressure of at least approximately 40 mm of Hg to help provide for smooth functioning of the urine collection device. Interruptions in suction line due to, for example, (i) transit phase for X-ray, CT scan or other imaging, (ii) malfunctioning vacuum pump, (iii) accidentally turning off vacuum switch, (iv) vacuum leak at canister, or (v) full canister may lead to lack of urine flow away from the urine collection device. In patients with urinary incontinence, such lack of urine flow due to interruption in the suction line may lead to collection of urine on fragile skin areas, presenting incontinence-associated dermatitis (IAD) risk.

In some examples, the present application provides for systems, devices, and methods that can help mitigate urine contacting the skin in the event of an interruption in the flow of urine along at least a portion of the suction line. For instance, in some examples of the present disclosure, a system can include (i) a first fluid collection device that can be coupled to a user and collect urine discharged from a body of the user and (ii) a second fluid collection device that can provide a safety feature to collect and "trap" urine away from the skin only if a vacuum interruption occurs. In particular, within examples, a responsive in-line urine collection system is disclosed to reduce or prevent collection of urine around skin areas in the absence of an external vacuum source and/or when a pressure of the vacuum falls below a threshold magnitude of pressure (e.g., 40 mm of Hg vacuum).

The present application additionally or alternatively provides for a urine collection device that can help to mitigate blockages of fluid flow between the urine collection device and the fluid collection reservoir coupled to the urine collection device by a drain tube. There are a number of possible causes of blockages of fluid flow in existing urine collection devices. For instance, some existing urine collection devices include a collection member that is formed from a relatively thin and flexible material, and a drain tube that is formed from a relatively more rigid material (e.g., as compared to the material of the collection member). In this arrangement, when greater than a threshold magnitude of vacuum pressure is applied to the drain tube and the collection member, the collection member may collapse on itself creating a potential blockage for fluid flow. Further, during normal use conditions, the device may get twisted at the interface of the drain tube and the collection member, and this may inhibit fluid flow. In addition, while clinicians take care to ensure that the drain tube is not blocked or tangled, there may be a risk that external objects (such as movement of medication transport trolley or clinician stepping on to the drain tube) or patient's own weight may block the drain tube.

In some examples, to help mitigate potential blockage issues, the present disclosure additionally or alternatively provides for a urine collection device that includes a spacer having a flat profile, pliable flow channel that may transmit vacuum using multiple channels. In one implementation, the spacer can provide the channels via an embossed pattern across an oval shape (instead of one single channel such as tubing with a circular cross-section). The spacer can provide for improved functionality as well as cost reduction relative to spacers used in some existing urine collection devices (e.g., spacers formed from polyester foam). The use of the spacer having a relatively wide, flat profile along with an embossed pattern (i.e., island of raised areas) helps ensure that (i) walls of the collection member do not collapse under relatively high vacuum pressures, (ii) twisting motion of the drain tube and/or the collection member still provides fluid flow due to presence of multiple flow channels, and (iii) even if some portions of the urine collection device are partially blocked due to patient's weight, additional flow channels can provide continuous flow of urine away from the fragile skin into the vacuum collection reservoir.

In other examples, to help mitigate potential blockage issues, the urine collection device can additionally or alternatively include a port support structure at an interface between the collection member and the drain tube. The port support structure can be formed from a material having a rigidity that is greater than a rigidity of the collection member. Additionally, the port structure can have a relatively flat and wide shape. The rigidity and/or the shape of the port support structure can help to reduce or prevent (i) collapse of the collection member due to vacuum pressure, (ii) kinking at the interface between the collection member and the drain tube, and/or (ii) closing of the of the fluid flow path due to external pressure applied to interface between the collection member and the drain tube.

In still other examples, to help mitigate potential blockage issues, the urine collection device can additionally or alternatively include a port for coupling the collection member to the drain tube, and the port can rotate relative to the collection member. In this arrangement, when the drain tube is moved relative to the collection member, the port can move with the drain tube such that forces applied by the drain tube to the collection member are reduced (e.g., as compared to implementations in which the port does not rotate relative to the collection member). As a result, the port being rotatable can help to mitigate the collection member twisting when the drain tube is moved relative to the collection member.

In additional or alternative examples, the present disclosure provides for a urine collection device that can help to reduce or prevent urine leaking out a proximal end of the collection member. For instance, the proximal end of the collection member can include an opening that provides access to an internal cavity of the collection member. In use, a user's penis can be received through the opening in the internal cavity. In one example, to mitigate leakage out of the opening at the proximal end, the collection member can include a plurality of tabs that can be adjusted to reduce a size of the opening after the user's penis is received through the opening in the internal cavity. In other example, the collection member can additionally or alternatively include a sealed edge that extends inwardly from lateral sides of the collection member such that the opening extends along only a portion of the proximal end (and not an entirety of the proximal end). Additionally or alternatively, the urine collection device can include an absorbent material at the proximal end in the internal cavity.

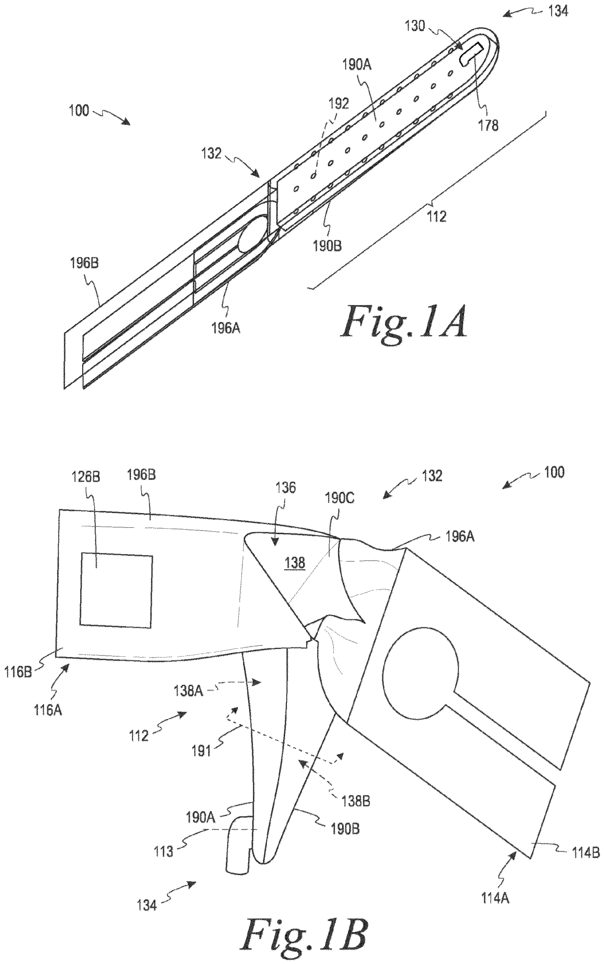

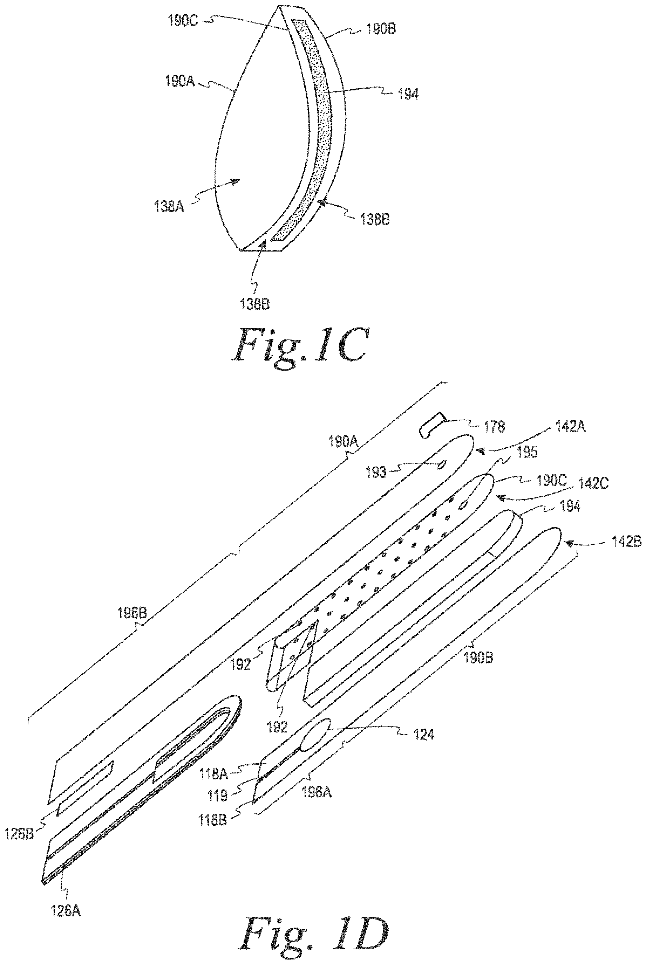

Referring now to Figures 1A-1D, a urine collection device 100 according to an example. More specifically, Figure 1A depicts a perspective view of the urine collection device 100, Figure 1B depicts another perspective view of the urine collection device1, Figure 1C depicts a cross-sectional view of the urine collection device 100 taken through a line 191 in Figure 1B, and Figure 1D depicts an exploded view of the urine collection device 100. As shown in Figures 1A-1D, the urine collection device 100 includes a collection member 112 that is suitable to direct urine to an outlet 130 of the collection member 112. The collection member 112 extends from a proximal end 132 to a distal end 134. As shown in Figures 1B-1C, the proximal end 132 includes an opening 136 that provides access to an internal cavity 138 of the collection member 112.

As also shown in Figures 1A-1D, the collection member 112 includes a top wall 190A, a bottom wall 190B, and inner wall 190C between the top wall 190A and the bottom wall 190B. In this arrangement, the internal cavity 138 includes a first chamber 138A and a second chamber 138B with the inner wall 190C separating the first chamber 138A from the second chamber 138B. As shown in Figures 1B-1C, the opening 136 in the collection member 112 is defined by the inner wall 190C and the top wall 190A of the collection member 112. Whereas, at the proximal end 132, the inner wall 190C is coupled to the bottom wall 190B of the collection member 112. As such, the inner wall 190C inhibits access to the second chamber 138B from outside of the first chamber 138A. Thus, when the user inserts a penis through the opening 136, the penis is received in the first chamber 138A of the internal cavity 138.

With the user's penis extending through the opening 136, urine can be initially received in the first chamber 138A of the collection member 112. The received urine can be directed from the first chamber 138A to the second chamber 138B so as to reduce (or minimize) contact between the user and the urine. This can beneficially improve user comfort, and/or improve hygiene and sanitary conditions for the user.

To direct the urine from the first chamber 138A to the second chamber 138B, the first chamber 138A is in communication (i.e., fluid communication) with the second chamber 138B. In one example, the inner wall 190C can be configured to provide for fluid communication between the first chamber 138A and the second chamber 138B. For instance, the inner wall 190C can define a plurality of perforations 192 through which the first chamber 138A can communicate with the second chamber 138B. In Figures 1A and 1D, the perforations 192 each have a generally circular shape; however, one or more of the perforations 192 can have a different shape in other example embodiments. Additionally, in Figures 1A and 1D, the perforations 192 are arranged in a pattern including three lines of perforations 192 in the inner wall 190C; however, the perforations 192 can be arranged in a different pattern in other example embodiments.

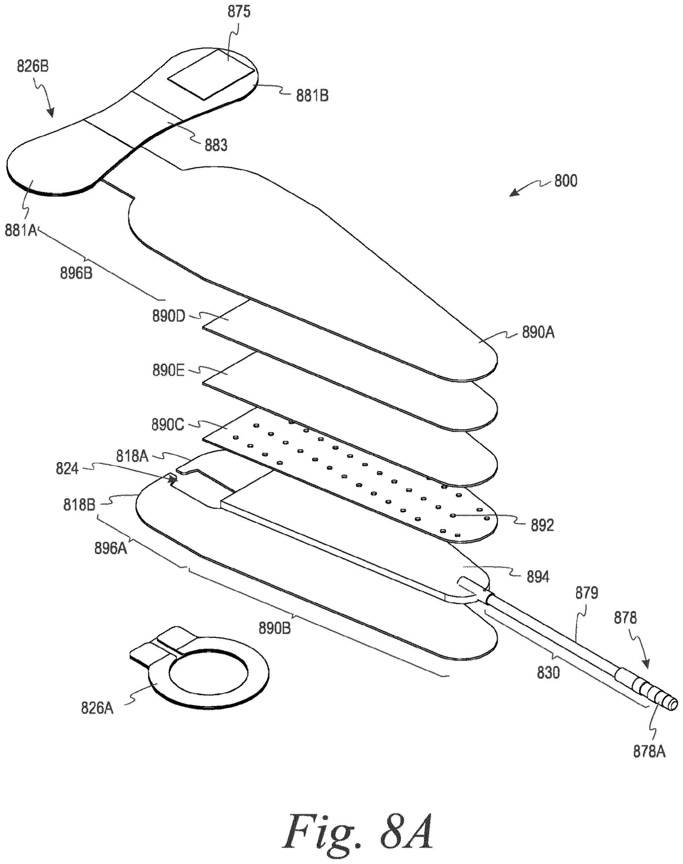

In some examples, the inner wall 190C can be formed from a fluid impervious material (e.g., a silicone, rubber, latex, a thermoplastic, and/or a polyurethane film), which defines the plurality of perforations 192. In other examples, the inner wall 190C can additionally or alternatively include a permeable layer and a wicking layer as described, for instance, in greater detail below with respect to Figures 8A-8D.

Additionally, to assist in directing the urine in the second chamber 138B to the outlet 130 at the distal end 134, the urine collection device 100 can include a spacer 194 in the second chamber 138B. The spacer 194 can, for example, help to separate the bottom wall 190B from the inner wall 190C. This can help to inhibit (or prevent) an occlusion of the second chamber 138B, which may negatively impact egress of the urine from the urine collection device 100. Additionally, for example, the spacer 194 can help to inhibit (or prevent) an occurrence of a vacuum lock condition due to the vacuum pressure applied by a vacuum device.

In one example, the spacer 194 is an open-cell foam material configured to allow the urine to flow through the spacer 194 in a direction from the proximal end 132 to the distal end 134. In another example, the spacer 194 can be made from other materials such as, for instance, a closed foam material and/or a batting material. However, an open-cell foam material can be particularly beneficial in that an open-cell foam material can allow the urine to flow both through and around the spacer 194. Whereas, other materials may limit and/or prevent the urine from flowing through the spacer 194. In some implementations, the open-cell foam material of the spacer 194 can allow the urine in the second chamber 138B to flow to the outlet 130 even when the spacer 194 is compressed.

In one implementation, for instance, the spacer 194 can include a thermally reticulated 45 ppi polyether polyurethane foam (PUR). Additionally, for instance, the spacer 994 can have (i) a pore size (visual) of approximately 40 pores per inch (ppi) to approximately 50 ppi, (ii) a density of approximately 1.3 pounds per cubic foot (lbs/ft³) to approximately 1.6 lbs/ft³, (iii) a tensile strength of at least approximately 10.0 pounds per square inch (psi), (iv) an elongation of 100 percent, (v) a tear of at least approximately 2.5 pounds per inch (lbs/in), (vi) a compression load deflection at 25% R (CLD 25%R(2"x2"x1")) of at least approximately 0.35 psi, (vii) a CLD 65%R(2"x2"x1") of at least approximately 0.43 psi, and/or (viii) a maximum compression set of approximately 15%.

In another example, for instance, the spacer 194 can include a plurality of gas filled pockets separated by gaps, which allow the urine to flow through and around the spacer 194. For example, the pockets can be similar to bubble wrap (e.g., made from (e.g., polyethylene pockets filled with air). A quantity, a size, and/or a density of the pockets can be arranged to allow for urine flow to the outlet 130 while inhibiting a vacuum lock condition when a vacuum pressure is applied to the urine collection device 100. In a further example, the spacer 194 can be constructed with a plurality of channels to allow the urine to flow to the outlet 130 while also allowing for air to flow, alleviating a vacuum lock condition. In one implementation, the spacer 194 can be made from a polymer material and molded, for example, by an injection molding or thermoformed molding process. Other examples are also possible.

As noted above, the collection member 112 can be made from a flexible material such as, for instance, silicone, rubber, latex, and/or a thermoplastic. The spacer 194 can also beneficially assist in providing support to the urine collection device 100 to, for instance, inhibit (or prevent) twisting of the collection member 112 and/or improve handling of the urine collection device 100. In Figures 1A and 1D, the spacer 194 extends from the proximal end 132 to the distal end 134. This can allow the spacer 194 to help to support the collection member 112 over a relatively large portion of the urine collection device 100. However, in other examples, the spacer 194 can extend over a smaller portion of the second chamber 138B.

As noted above, the collection member 112 is suitable to (i) direct urine from the first chamber 138A to the second chamber 138B (e.g., via the perforations 192) and (ii) direct the urine in the second chamber 138B distally toward the outlet 130 (e.g., through and/or around the spacer 194). The outlet 130 is configured to egress the urine from internal cavity 138 of the collection member 112. For example, the outlet 130 can include an aperture 193 in the top wall 190A and a port 178, which is suitable to be coupled to a drain tube. The port 178 can be coupled to the top wall 190A at the aperture 193 by, for example, radiofrequency (RF) heat sealing and/or RF welding.

Although the aperture 193 is formed in the top wall 190A in Figures 1A-1D, the aperture 193 can be formed in the bottom wall 190B in another example. In this alternative example, the port 178 can also be coupled to the bottom wall 190B at the aperture 193. This can help to facilitate draining and suctioning liquid from the collection member 112. By contrast, positioning the aperture 193 and the port 178 at the top wall 190A can make the port 178 more easily accessible than positioning the port 178 at the bottom wall 190B.

In some examples, the port 178 can be fixedly coupled to the collection member 112 such that the port 178 is not rotatable relative to the collection member 112. This can simplify a manufacturing process and/or reduce a cost of manufacture. In such examples, the port 178 can have a single configuration and orientation relative to the collection member 112. For instance, the port 178 can include a bend that orients an outtake of the port 178 in the distal direction. This can help to direct the drain tube in the distal direction away from the user. In another implementation, the port 178 can include a bend that orients the outtake of the port 178 in the proximal direction. In instances in which a vacuum device and/or a fluid collection reservoir are at a location that is proximal to the port 178, this can help to mitigate bending a drain tube coupling the port 178 to the vacuum device and/or a fluid collection reservoir. In other implementations, the port 178 can be oriented in other directions (e.g., transverse to a longitudinal axis of the collection member 112).

In other examples, the port 178 can be rotatably coupled to the collection member 112 such that port 178 can rotate about an axis relative to the collection member 112. For instance, as shown in Figure 1B, the port 178 can include a joint that is configured to allow the port 179 to rotate about an axis 113. The axis 113 can extend through the aperture 193. In this arrangement, rotation of the port 178 can help to inhibit twisting or kinking of the collection member 112 and/or the drain tube when the drain tube and/or the collection member 112 are moved relative to each other. As a result, the port 178, which is rotatable, can help to mitigate blockage of fluid flow from the collection member 112 to the port 178 and the drain tube.

In some implementations, the port 178 is freely rotatable by more than 360 degrees relative to the collection member 112. This can help mitigate twisting and kinking in all rotational positions of the port 178 relative to the collection member 112. However, in other implementations, the port 178 can be rotatable by less than 360 degrees relative to the collection member 112. This may help to constrain the drain tube to a particular range of positions relative to the collection member 112, which may beneficially help to maintain the drain tube at a position extending away from the user.

In one example, the port 178 can include a thread for threadably coupling with the drain tube. In another example, the port 178 can include a hose barb and/or a Luer lock for coupling with the drain tube. In another example, as described in further detail below, the port 178 can include a tapered end portion for coupling with the drain tube. Given that drain tubes may have different sizes (e.g., depending on the make and/or model of the vacuum device), the tapered end portion of the port 178 can help to more universally couple the port 178 to a plurality of differently sized drain tubes.

In Figures 1A-1D, the outlet 130 is at a location spaced away from the distal end 134. As described in further detail below, the outlet 130 can be at other locations such as, for example, at a distalmost point of the collection member 112 in other examples. By locating the outlet 130 near or at the distal end 134, pooling of urine at the distal end 934 can be reduced (or minimized).

As noted above, the collection member 112 is suitable to (i) direct urine from the first chamber 138A to the second chamber 138B and (ii) direct the urine in the second chamber 138B distally toward the outlet 130. To facilitate egressing the urine in the second chamber 138B from the collection member 112, the inner wall 190C can include a passage 195 at the outlet 130. The passage 195 can be similar to the perforations 192 in the inner wall 190C in that the passage 195 provides for communication between the second chamber 138B and the first chamber 138A. In one example, the passage 195 can have a size that is greater than a size of each of the perforations 192. Additionally, the passage 195 can be aligned with outlet 130 of the collection member 112 (e.g., aligned with the aperture 193 in the top wall 190A). In this arrangement, when the vacuum device applies the vacuum pressure to the outlet 130, the urine can be directed under suction (i) from the second chamber 138B to the first chamber 138A via the passage 195 (and/or adjacent ones of the perforations 192), and (ii) from the first chamber 138A into the drain tube via the outlet 130.

Although the passage 195 is larger than each perforation 192 in Figures 1A-1D, the passage 195 can have a size that is the same as and/or smaller than one or more of the perforations 192 in other examples. However, providing the passage 195 with a size that is larger than the perforations 192 can help to receive and/or maintain the urine in the second chamber 138B in areas proximal to the outlet 130 (e.g., as the urine is flowing in a direction from the proximal end 132 toward the distal end 134).

Additionally, as noted above, the passage 195 is aligned with the outlet 130 in Figures 1A-1D. This can, for example, help to apply the vacuum pressure more directly to the urine in the second chamber 138B when the outlet 130 is on the top wall 190A. However, the passage 195 can be at other locations relative to the outlet 130 in other examples. Further, although the passage 195 is distal of the perforations 192 in Figures 1A-1D, one or more of the perforations can be distal of the passage 195 in other examples.

As described above, when the user inserts a penis through the opening 136, the penis is received in the first chamber 138A of the internal cavity 138. Within examples, the first chamber 138A of the collection member 112 can have a size and shape that provides for the collection member 112 loosely enveloping the penis when inserted through the opening 136. For instance, in one implementation, at least a portion of the collection member 112 at the first chamber 138A can have a circumference, which is greater than most (or all) penis sizes. By loosely enveloping the penis, the collection member 112 can reduce (or minimize) discomfort of the user relative to condom catheters, for example.

As shown in Figures 1A-1D, the urine collection device 100 further includes one or more attachment members 196A-196B that are suitable to secure the urine collection device 100 to a user. In particular, in Figures 1A-1D, the urine collection device 100 includes a first attachment member 196A and a second attachment member 196B. However, the urine collection device 100 can include a single attachment member 196A-196B or more than two attachment members 196A-196B in other examples.

The first attachment member 196A extends from the bottom wall 190B of the collection member 112 at the proximal end 132. The first attachment member 196A includes an outer side 114A for contacting a pelvic area of a user and an inner side 114B facing the collection member 112 when the urine collection device 100 is secured to the user. The first attachment member 196A also defines an aperture 124, which extends through the first attachment member 196A from the outer side 114A to the inner side 114B. When the urine collection device 100 is secured to the user, the first attachment member 196A receives the penis of the user in the aperture 124 with the outer side facing the pelvic area of the user and the inner side facing the collection member 112.

As shown in Figure 1D, the first attachment member 196A also includes a plurality of flexible members 118A-118B that facilitate access to the aperture 124. Specifically, in Figure 1D, the first attachment member 196A includes a first flexible member 118A and a second flexible member 118B that are movable relative to each other to facilitate access to the aperture 124 in the first attachment member 196A. For example, as shown in Figure 1D, the first flexible member 118A can be separated from the second flexible member 118B by a slit 119 extending distally from the aperture 924 in the first attachment member 196A. In Figure 1D, the slit 119 extends from the aperture 124 to a distalmost point of the first attachment member 196A. However, the slit 119 can extend to a point that is proximal of the distalmost point of the first attachment member 196A in another example embodiment.

In this arrangement, to receive the penis through the aperture 124, the first flexible member and the second flexible member can be deflected away from each other to expand the size of the aperture 124. After the aperture 124 is expanded, the first attachment member 196A can be positioned on the user with the penis extending through the aperture 124. After the first attachment member 196A is positioned on the user such that the penis extends through the aperture 124, the first flexible member 118A and the second flexible member 118B can be moved back toward each other to reduce the size of the aperture 124 to a size that comfortably fits around the penis. As such, the first flexible member 118A and the second flexible member 118B can be manipulated to adjust the size of the aperture 124 and thereby accommodate a specific size and/or shape of a given user's penis.

In some examples, when the penis is inserted through the aperture 124, the first flexible member 118A and the second flexible member 118B can additionally apply a force on the penis that assists in retaining the penis in a desired position relative to the collection member 112. As the size of the aperture 124 can expand to an extent commensurate with the size of the particular user's penis, the first attachment member 196A can provide a more universal fit for a relatively broad range of the male population.

The first attachment member 196A can include a first adhesive 126A to further assist in securing the first attachment member 196A to a user. For instance, the first adhesive 126A can be coupled to the outer side 114A of the first attachment member 196A. As such, when the first attachment member 196A is secured to the user, the first adhesive 126A on the outer side 114A of the first attachment member 196A can contact and adhere to the pelvic area of the user to assist in retaining the urine collection device 100 in the desired position. By providing the first adhesive 126A on the outer side 114A, which contacts the pelvic area of the user, the first adhesive 126A can more comfortably adhere the urine collection device 100 to a less sensitive part of the body than a condom catheter.

As shown in Figures 1A and 1D, the first adhesive 126A can have a shape that generally corresponds to a shape of the first attachment member 196A. For instance, the first adhesive 126A can extend around at least a portion of the aperture 124, along at least a portion of the first flexible member 118A, and along at least a portion of the second flexible member 118B. This can help to secure the first attachment member 196A to the user over a relatively large surface area of the first attachment member 196A, which can help to maintain the urine collection device 100 in a desired position relative to the user. Also, in some examples, the first adhesive 126A can extend entirely around the aperture 124 in the first attachment member 196A.

As shown in Figures 1A, 1B, and 1D, the second attachment member 196B extends from the top wall 190A of the collection member 112 at the proximal end 132. The second attachment member 196B includes an inner side 116B for contacting a pelvic area of a user and an outer side 116A facing away from the user when the urine collection device 100 is secured to the user. Additionally, the second attachment member 196B includes a second adhesive 126B to assist in securing the second attachment member 196B to the user. Specifically, the second adhesive 126B is coupled to the inner side 116B of the second attachment member 196B.

In some examples, the second attachment member 196B can have a length (i.e., a dimension along a longitudinal axis) that is greater than a length of the first attachment member 196A. This can assist in allowing the second attachment member 196B to be secured to the user at a location that is above a location at which the first attachment member 196A is secured to the user.

As shown in Figure 1D, the urine collection device 100 can include a plurality of sheets 142A-142C of material. In particular, the urine collection device 100 can include a first sheet 142A, a second sheet 142B, and a third sheet 142C, which are coupled to each other to form the urine collection device 100. As examples, the sheets 142A-142C can be coupled to each other by, for instance, radio frequency (RF) heat sealing and/or RF welding.

The first sheet 142A can provide the top wall 190A of the collection member 112 and the first attachment member 196A, and the second sheet 142B can provide the bottom wall 190B and the second attachment member 196B of the collection member 112. The third sheet 142C is between the first sheet 142A and the second sheet 142B.

In this arrangement, the third sheet 142C can provide the inner wall 190C that divides the internal cavity 138 into the first chamber 138A and the second chamber 138B. Specifically, the first chamber 138A can be defined by a space between the first sheet 142A and the second sheet 142B (i.e., between the top wall 190A and the inner wall 190C), whereas the second chamber 138B can be defined by a space between the second sheet 142B and the third sheet 142C (i.e., between the inner wall 190C and the bottom wall 190B).

In Figure 1D, the first attachment member 196A is integrally formed with the top wall 190A and the second attachment member 196B is integrally formed with the bottom wall 190B. This can beneficially reduce (or minimize) a risk of the first attachment member 196A and/or the second attachment member 196B becoming detached from the collection member 112. However, in other examples, the first attachment member 196A can be coupled to the top wall 190A and/or the second attachment member 196B can be coupled to the bottom wall 190B.

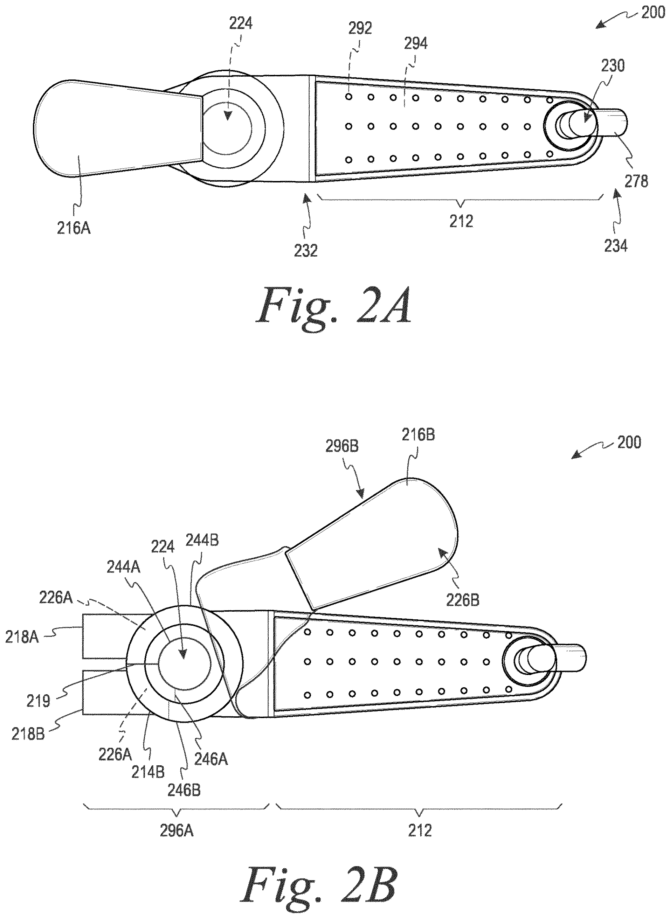

Referring now to Figures 2A-2C, a urine collection device 200 is depicted according to another example. The urine collection device 200 is substantially similar to the urine collection device 100 illustrated and described with respect to Figures 1A-1D, except the urine collection device 200 includes attachment members that differ in some ways from the first attachment member 196A and the second attachment member 196B in Figures 1A-1D.

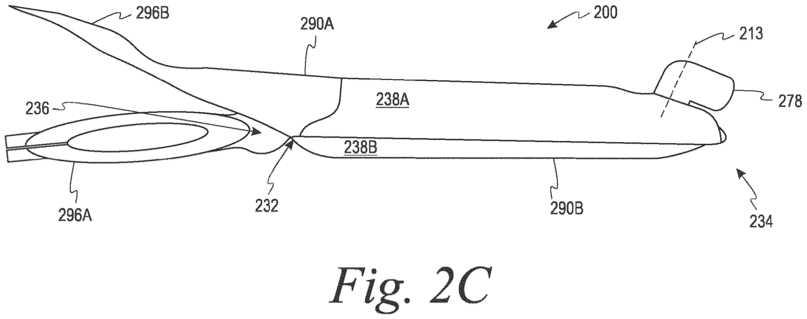

As shown in Figures 2A-2C, the urine collection device 200 includes a collection member 212 extending from a proximal end 232 to a distal end 234. As shown in Figures 2A-2B, the collection member 212 has a width that tapers inwardly along a longitudinal axis from the proximal end 232 to the distal end 234. As shown in Figure 2C, the proximal end 232 includes an opening 236 that provides access to an internal cavity 238, which has a first chamber 238A in communication (i.e., fluid communication) with a second chamber 238B. The urine collection device 200 includes an inner wall separating the first chamber 238A from the second chamber 238B and the inner wall includes a plurality of perforations 292 through which the first chamber 238A communicates with the second chamber 238B. As described above, the inner wall can be configured differently in other examples (e.g., including a permeable layer and a wicking layer). A spacer 294 is in the second chamber 238B. Additionally, the urine collection device 200 includes an outlet 230 for egressing urine from the internal cavity 238 of the collection member 212. The outlet 230 includes a port 278 that is rotatable relative to the collection member 212 in Figures 2A-2C (e.g., rotatable about an axis 213). However, the port 278 can be rotationally fixed relative to the collection member 212 in other examples.

In Figures 2A-2C, a first attachment member 296A extends from a bottom wall 290B of the collection member 212 at the proximal end 232, and a second attachment member 296B extends from a top wall 290A of the collection member 212 at the proximal end 232. The first attachment member 296A includes an inner side 214B and an outer side opposite the inner side 214B in Figure 2B, and the second attachment member 296B includes an outer side 216A and an inner side 216B. The first attachment member 1096A also includes a first adhesive 1026A on the outer side of the first attachment member 296A (i.e., on the side opposite the inner side 214B shown in Figure 2B), and the second attachment member 296B includes a second adhesive 226B on the inner side 216B of the second attachment member 296B to assist in securing the urine collection device 200 to a user.

As shown in Figure 2B, the first attachment member 296A includes a first flexible member 218A and a second flexible member 218B. The first flexible member 218A and the second flexible member 218B of the first attachment member 296A define an aperture 224 in the first attachment member 296A. As shown in Figure 2B, the first flexible member 218A and the second flexible member 218B are separated by a slit 219 and are independently movable relative to each other (and/or the collection member 212).

As shown in Figure 2B, the first attachment member 296A includes a ring-shaped portion extending between an inner edge 244A and an outer edge 244B. The inner edge 244A defines a circumference of the aperture 224 in the first attachment member 296A, and outer edge 244B defines a circumference of the ring-shaped portion of the first attachment member 296A. In this arrangement, the first flexible member 218A and the second flexible member 218B can each define a respective semi-circular arc.

In one example, the inner edge 244A can be a circle having a diameter of approximately 20 millimeters (mm) and the outer edge 244B can have a diameter of approximately 40 mm. This can allow for a size of the aperture 224 of the first attachment member 296A to be dynamically adjusted within a range of sizes between approximately 20 mm and 40 mm based on, among other things, a position of the first flexible member 218A and the second flexible member 218B relative to each other. As explained below, the aperture 224 can be dynamically expanded to accommodate penises having different sizes and/or shapes and thereby provide a more universal fit than existing urine collection devices (e.g., condom catheters). For instance, to accommodate penises having sizes between 20 mm and 40 mm, conventional condom catheters are typically required to come in five or more different sizes (e.g., a 21 mm size catheter, a 25 mm size catheter, a 28 mm size catheter, a 30 mm size catheter, a 35 mm size catheter, and a 40 mm size catheter).

As also shown in Figure 2B, the ring-shaped portion of the first attachment member 296A further includes an inner portion 246A and an outer portion 246B. The first adhesive 226A is coupled to the outer portion 246B, and the inner portion 246A extends from the outer portion 246B to the inner edge 244A. In some examples, the first adhesive 226A can include a stiffening member that enhances the rigidity of a portion of the first attachment member 296A coupled to the first adhesive 226A. For instance, the first adhesive 226A can include a flexible cushion, which can enhance the rigidity of the outer portion 246B of the first attachment member 296A and/or improve user comfort. In other examples, the first adhesive 226A can omit the stiffening member such that the first adhesive 226A does not materially affect the rigidity of the first attachment member 296A. This can help the first attachment member 296A better conform to a particular anatomy of a user and enhance a surface area of the coupling between the first attachment member 296A and the user (e.g., as compared to examples in which the first adhesive 226A includes the stiffening member).

In this arrangement, the first attachment member 296A is configured such that the inner portion 246A can deflect relative to the outer portion 246B. This can beneficially provide for the inner portion 246A deflecting distally relative to the outer portion 246B when the user's penis is inserted and/or received through the aperture 224, and thereby expanding the size of the aperture 224 to accommodate differently sized and shaped penises. The relatively flexible inner portion 246A can additionally or alternatively improve patient comfort by reducing (or minimizing) an amount of pressure applied by the first attachment member 296A to the penis.

In the example shown in Figures 1A-1D, the first adhesive 126A extends proximally from the first attachment member 196A. By contrast, in Figures 2A-2C, the first adhesive 226A is generally co-extensive with the first attachment member 296A.

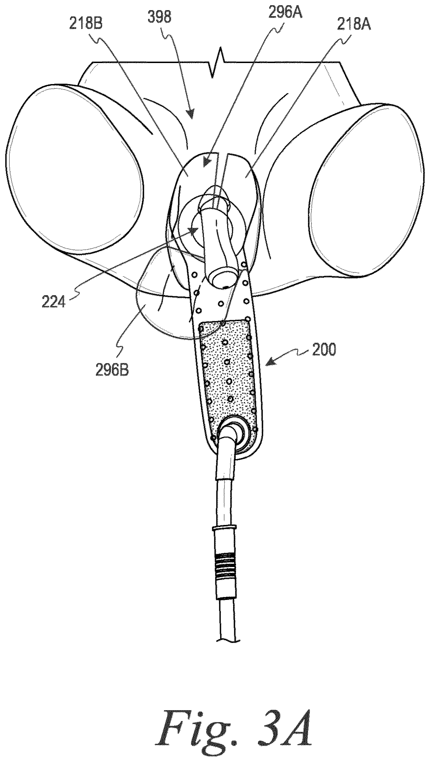

Referring now to Figures 3A-3C, a process for using the urine collection device 200 is depicted according to an example embodiment. To secure the urine collection device 200 to a user, the first flexible member 218A and the second flexible member 218B are moved away from each other to expand the size of the aperture 224 in the first attachment member 296A. Next, the first attachment member 296A is positioned on a pelvic area 398 of the user such that the penis of the user extends through the aperture 224. For instance, in one implementation, the first attachment member 296A can be first moved to a position directly below the penis and then, while the aperture 224 is expanded, raised until the penis is received in the aperture 224. That is, the penis can pass through the slit 219 between the first flexible member 218A and the second flexible member 218B to position the penis in the aperture 224. In another implementation, the first attachment member 296A can be positioned such that the aperture 224 is aligned with the penis and then the first attachment member 296A can be moved proximally to insert the penis through the aperture 224.

After the penis is received in the aperture 224, the first flexible member 218A and the second flexible member 218B can be moved toward each other to reduce the size of the aperture 224, and the first attachment member 296A can be coupled to the pelvic area 398 of the user by the first adhesive 226A. In this way, the first attachment member 296A can provide for dynamically adjusting the size of the aperture 224 to accommodate the specific size and/or shape of the penis, as described above.

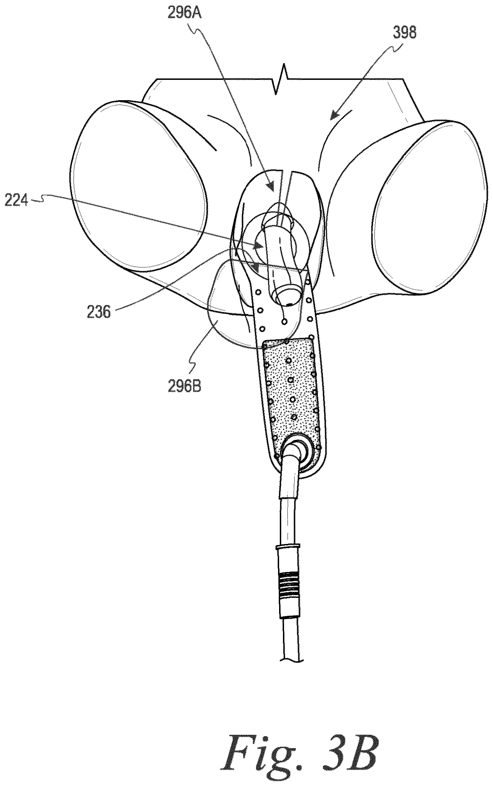

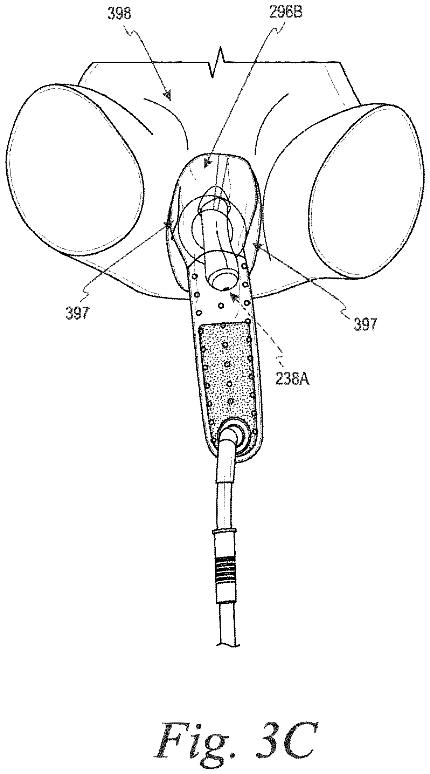

Figure 3A depicts the first attachment member 296A secured to the pelvic area 398 of the user with the penis of the user extending through the aperture 224 in the first attachment member 296A. Additionally, in Figure 3A, the penis is outside of the first chamber 238A of the collection member 212. Next, the second attachment member 296B can be moved so that the penis is inserted through the opening 236. Figure 3B depicts the urine collection device 200 with the user's penis extending through the aperture 224 of the first attachment member 296A and the opening 236 of the collection member 212 into the first chamber 238A of the collection member 212. As shown in Figure 3C, after the penis is inserted into the first chamber 238A, the second attachment member 296B is secured to the pelvic area 398 of the user by the second adhesive 226B.

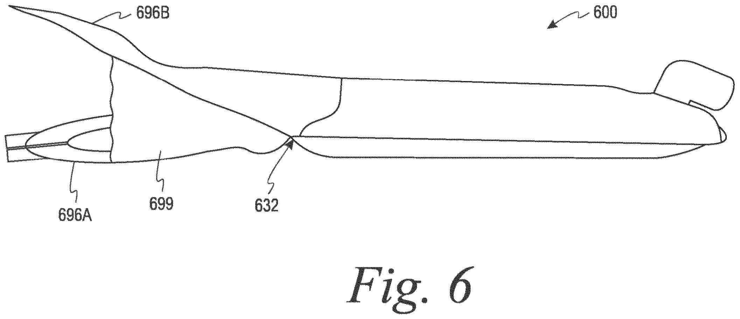

Additionally, as shown in Figure 3C, when the urine collection device 200 secured to the user, a gap 397 is formed between the first attachment member 296A and the second attachment member 296B on opposing sides of the urine collection device 200. The gaps 397 can beneficially allow for air flow through the opening 236 and into the first chamber 238A. This air flow can beneficially help to, for example, maintain sanitary conditions and/or improve user comfort. Additionally, for example, the airflow provided by the gaps can help to reduce (or minimize) a risk of a vacuum lock condition occurring. However, as described below with reference to Figures 6 and 16-17B, the urine collection device 200 can include one or more features for reducing or eliminating the gaps 397 to help mitigate leakage at the opening 136, 236 in other examples.

With the urine collection device 200 secured to the user as shown in Figure 3C, the user's penis is received in the first chamber 238A and above the second chamber 238B. When the user urinates, the urine is initially received in the first chamber 238A. The urine is then directed through the inner wall from the first chamber 238A to the second chamber 238B (e.g., via the perforations 292 in the inner wall). As the first chamber 238A is separated from the second chamber 238B by the inner wall, contact between the user's penis and the urine is reduced or minimized. Additionally, the spacer 1094 in the second chamber 1038B can assist in maintaining the penis at an elevated position relative to the urine in the second chamber 238B and, thus, can help to further reduce or minimize contact between the user's penis and the urine. By reducing or minimizing contact between the user's penis and the urine, the urine collection device 200 improves sanitary conditions, reduces the risk of infection, and/or improves user comfort.

The urine in the second chamber 238B can be directed distally toward the outlet 230. At the outlet 230, the urine can be egressed from the collection member 212, for example, under a vacuum pressure applied by a vacuum device and a drain tube coupled to the outlet 230 of the collection member 212. In implementations in which the port 278 is rotatable relative to the collection member 212, moving the drain tube relative to the collection member 212 can cause the port 278 to rotate relative to the collection member 212, which can help to inhibit blockage of urine flow through the outlet 230.

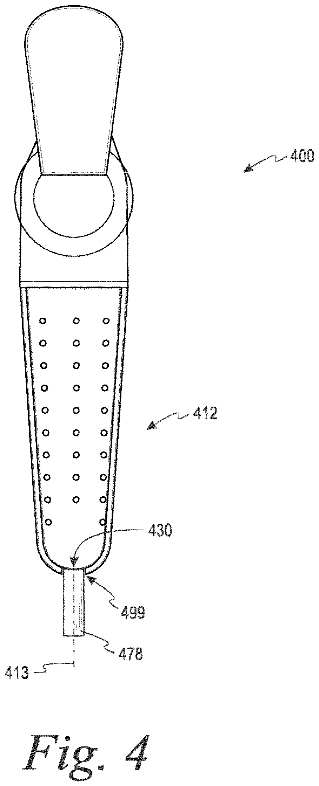

In Figures 1A-3C, the outlet 130, 230 is at a location on the top wall 190A, 290A spaced away from the distal end 134, 234 of the collection member 112, 212. However, as noted above, the outlet 130, 230 can be at a distalmost point of the collection member 112, 212 in other examples. Figure 4 depicts a urine collection device 400 including an outlet 430 at a distalmost point of a collection member 412. For example, the collection member 412 can be formed from a plurality of sheets of material (e.g., the first sheet 142A, the second sheet 142B, and/or the third sheet 142C), and the outlet 430 can include a port 478 coupled to the distalmost point of the collection member 412 at a seam 499 between the sheets of material. In implementation, the port 478 can be coupled to sheets of material by RF welding and/or RF heat sealing of the sheets around the port 478. By locating the outlet 430 at the distalmost point, pooling of urine in the collection member 412 can be reduced (or minimized) relative to locating the outlet at more proximal locations.

Additionally, as shown in Figure 4, because the port 478 is at the distalmost point of the collection member 412, the port 478 can have a generally linear shape that can egress urine in a direction that is generally parallel to a longitudinal axis of the collection member 412. Whereas, in Figures 1A-3C, the port 178, 278 includes an approximately 90 degree bend to facilitate directing the urine through the top wall 190A, 290A of the collection member 112, 212 to the direction that is generally parallel to the longitudinal axis (and away from the user).

In some examples, the port 478 can be rotatable relative to the collection member 412. For instance, the port 478 can be rotatable about a longitudinal axis 413 of the port 478. In other examples, the port 478 can be rotationally fixed relative to the collection member 412.

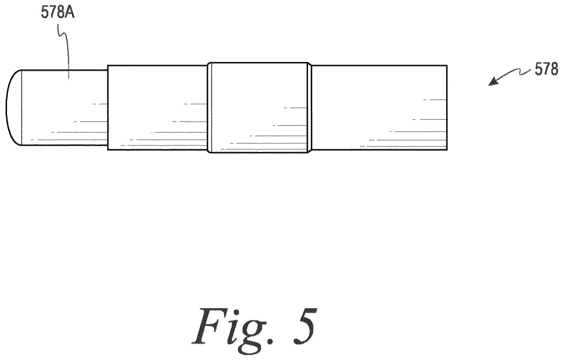

Additionally, as noted above, the port 178, 278, 478 of the urine collection device 100, 200, 400 can include a tapered end portion for coupling with the drain tube. For example, Figure 5 depicts a port 578 having a tapered end portion 578A according to another example embodiment. Given that drain tubes may have different sizes (e.g., depending on the make and/or model of the vacuum device), the tapered end portion of the port 1378 can help to more universally couple the port 1378 to a plurality of differently sized drain tubes.

As described above, the urine collection device 100, 200, 400 forms the gaps 397 between the first attachment member 196A, 296A and the second attachment member 196B, 296B when the urine collection device 100, 200, 400 is secured to the user. Additionally, as described above, the gaps 397 can beneficially allow for air flow through the opening 136, 236 and into the first chamber 138A, 238A. This air flow can beneficially help to, for example, maintain sanitary conditions and/or improve user comfort. Additionally, for example, the airflow provided by the gaps can help to reduce (or minimize) a risk of a vacuum lock condition occurring.

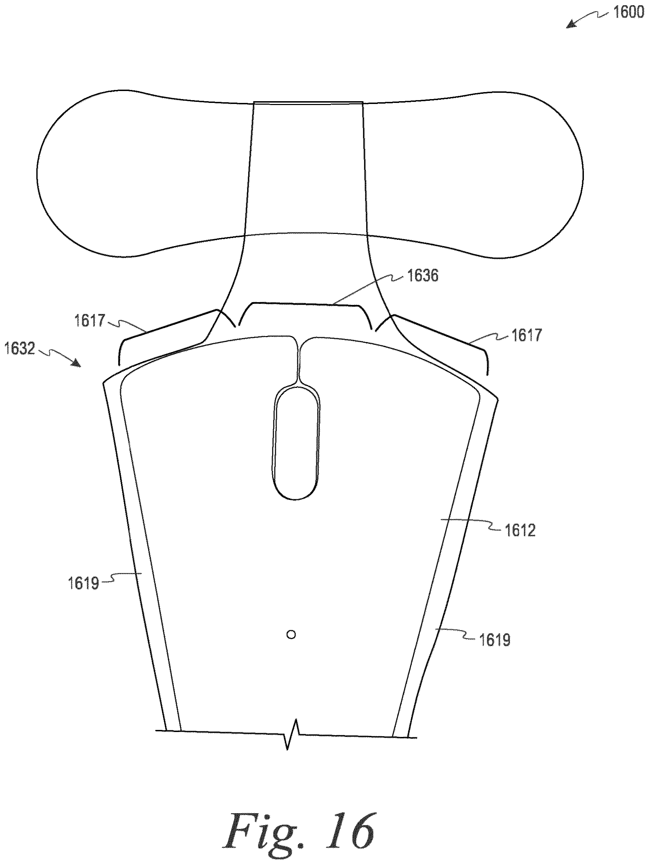

However, in another example, the urine collection device 100, 200, 400 can include one or more baffle portions (i.e., flange portions) that reduce (or eliminate) a size of the gaps 397 between the first attachment member 196A, 296A and the second attachment member 196B, 296B. As an example, Figure 6 depicts a urine collection device 600 that includes a first baffle portion 699 extending between a first attachment member 696A and the second attachment member 696B on a first lateral side and a second baffle portion 699 extending between the first attachment member 696A and the second attachment member 696B on a second lateral side. For user's with relatively small sized penises, the baffle portions 699 can help to retain the user's penis in the urine collection device 600 and/or reduce (or minimize) leakage of urine from a proximal end 632. Within examples, the baffle portions 699 can each have an accordion structure or a pleated structure that facilitates extending and retracting a size of the baffle portion 699. Additional or alternative approaches to mitigating leakage at the proximal end 632 are described in further detail below with respect to Figures 16-17B.

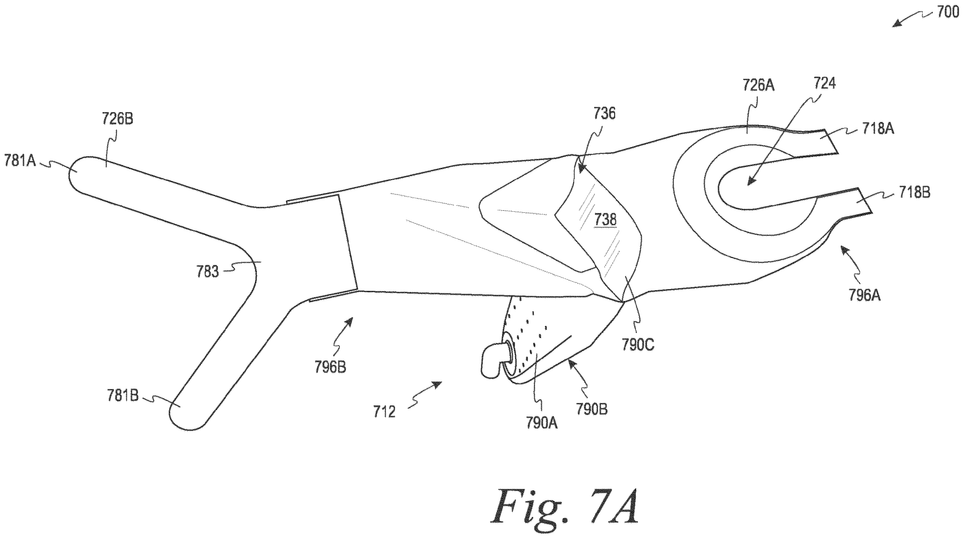

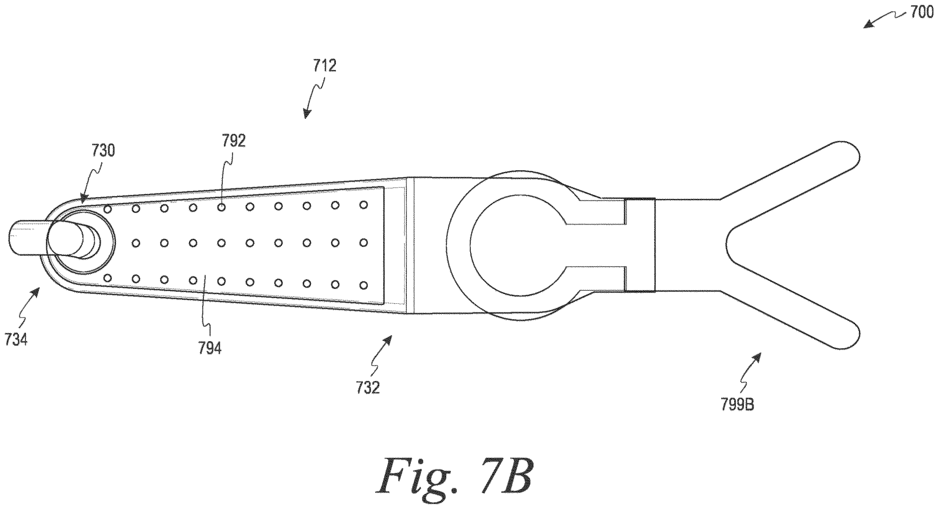

Referring now to Figures 7-7B, a urine collection device 700 is illustrated according to another example embodiment. The urine collection device 700 is substantially similar to the urine collection devices 100, 200, 400, 600 described above.

For example, as shown in Figures 7A-7B, the urine collection device 700 includes a collection member 712 extending from a proximal end 732 to a distal end 734. As shown in Figure 7B, the collection member 712 has a width that tapers inwardly along a longitudinal axis from the proximal end 732 to the distal end 734. As shown in Figure 7A, the proximal end 732 includes an opening 736 that provides access to an internal cavity 738, which has a first chamber in communication with a second chamber (e.g., the internal cavity 138, 238, which has the first chamber 138A, 238A in fluid communication with the second chamber 138B, 238B). The urine collection device 700 includes an inner wall 790C separating the first chamber from the second chamber and the inner wall 790C provides for fluid communication between the first and second chambers (e.g., the inner wall 790C can include a plurality of perforations 792 through which the first chamber communicates with the second chamber, and/or the inner wall 790C can include a permeable layer and a wicking layer). A spacer 794 is in the second chamber. Additionally, the urine collection device 700 includes an outlet 730 for egressing urine from the internal cavity 738 of the collection member 712.

In Figures 7A-7B, a first attachment member 796A extends from a bottom wall 790B of the collection member 712 at the proximal end 732, and a second attachment member 796B extends from a top wall 790A of the collection member 712 at the proximal end 732. The first attachment member 796A includes an inner side and an outer side opposite the inner side, and the second attachment member 796B includes an outer side and an inner side. The first attachment member 796A also includes a first adhesive 726A on the outer side of the first attachment member 796A, and the second attachment member 796B includes a second adhesive 726B on the inner side of the second attachment member 796B to assist in securing the urine collection device 700 to a user.

As shown in Figure 7A, the first attachment member 796A includes a first flexible member 718A and a second flexible member 718B. The first flexible member 718A and the second flexible member 718B of the first attachment member 796A define an aperture 724 in the first attachment member 796A. As shown in Figure 7A, the first flexible member 718A and the second flexible member 718B are separated and independently movable relative to each other (and/or the collection member 712).

The urine collection device 700 differs from the urine collection devices 100, 200, 400, 600 in that the second attachment member 796B includes a first arm 781A and a second arm 781B laterally extending from a center portion 783. More specifically, the first arm 781A and the second arm 781B laterally diverge from each other as the first arm 781A and the second arm 781B extend proximally from the center portion 783. In this arrangement, when the second attachment member 796B is secured to the user, the center portion 783 can be located at a middle area of the user's pelvic area and/or abdomen. Given that a relatively large portion of the male population has a greater density of hair near the middle area of the pelvic area and/or abdomen and a relatively lesser density of hair at areas adjacent to the middle area, the laterally-extending configuration of the second attachment member 796B can reduce (or minimize) contact between the second adhesive 726B and the user's hair. As such, the arrangement of the second attachment member 796B and the second adhesive 726B can improve patient comfort.

Additionally, because the first arm 781A and the second arm 781B laterally extend from the center portion 783, the first arm 781A and the second arm 781B can help to increase stability of the urine collection device 700 relative to urine collection devices that omit the first arm 781A and the second arm 781B laterally extending from the center portion 783.

In Figures 7A-7B, the second attachment member 796B is generally Y-shaped. As such, in Figures 7A-7B, the center portion 783 can be a vertex from which the first arm 781A and the second arm 781B laterally and proximally extend. In another example, the first arm 781A and the second arm 781B can laterally extend from the center portion 783 such that the second attachment member 796B is generally T-shaped. Other examples are also possible.

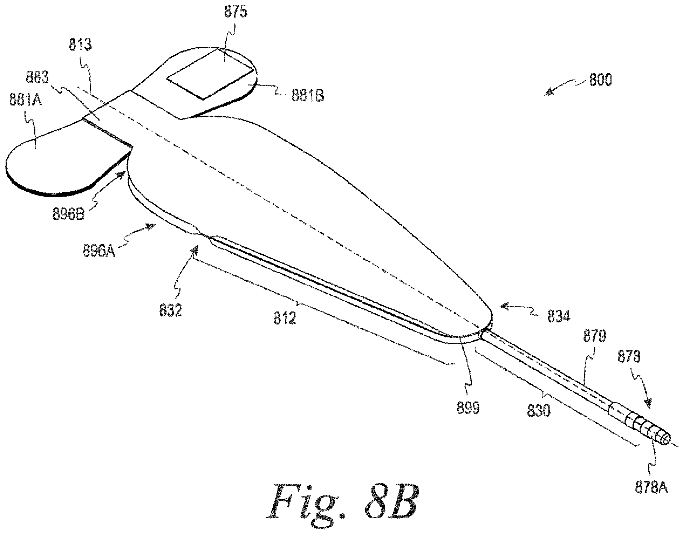

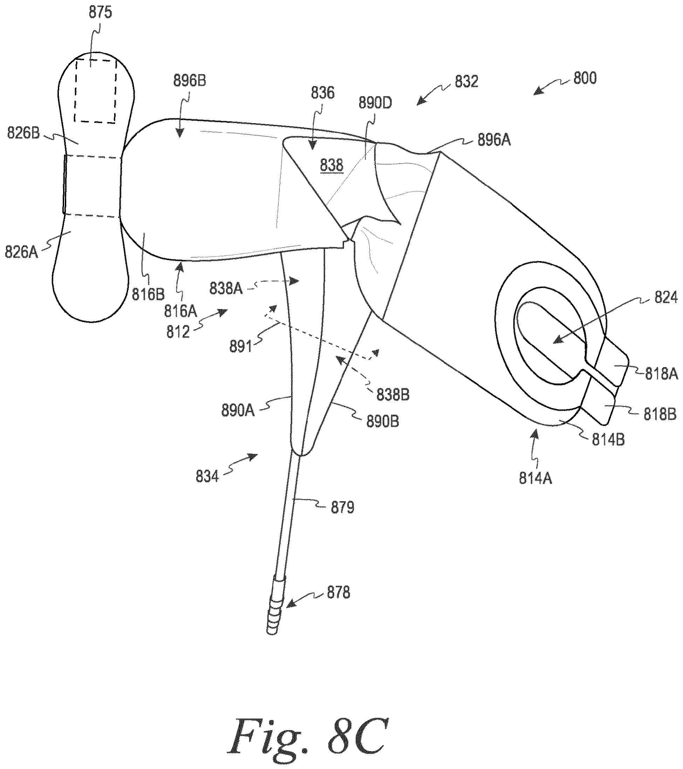

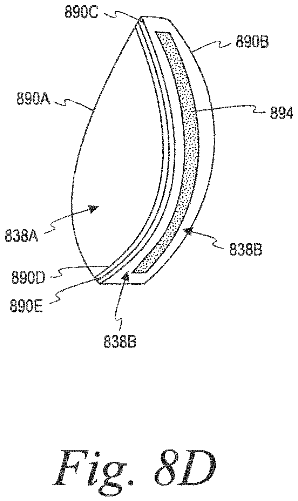

Referring now to Figures 8A-8D, a urine collection device 800 is illustrated according to another example embodiment. More specifically, Figure 8A is an exploded view of the urine collection device 800, Figure 8B is a perspective view of the urine collection device 800, Figure 8C is another perspective view of the urine collection device 800, and Figure 8D depicts a cross-sectional view of the urine collection device 800 taken through a line 891 in Figure 8C. The urine collection device 800 is substantially similar to the urine collection devices 100, 200, 400, 600, 700 described above.

For example, as shown in Figures 8B-8C, the urine collection device 800 includes a collection member 812 extending from a proximal end 832 to a distal end 834. As shown in Figure 8B, the collection member 812 has a width that tapers inwardly along a longitudinal axis 813 from the proximal end 832 to the distal end 834.

Additionally, in Figures 8A-8D, a first attachment member 896A extends from a bottom wall 890B of the collection member 812 at the proximal end 832, and a second attachment member 896B extends from a top wall 890A of the collection member 812 at the proximal end 832. The first attachment member 896A includes an inner side 814B and an outer side 814A opposite the inner side 814B, and the second attachment member 896B includes an outer side 816A and an inner side 816B. The first attachment member 896A also includes a first adhesive 826A on the outer side 814A of the first attachment member 896A, and the second attachment member 896B includes a second adhesive 826B on the inner side 816B of the second attachment member 896B to assist in securing the urine collection device 800 to a user.

Figures 8A and 8C depict the first attachment member 896A including a first flexible member 818A and a second flexible member 818B. The first flexible member 818A and the second flexible member 818B of the first attachment member 896A define an aperture 824 in the first attachment member 896A. As shown in Figure 8A, the first flexible member 818A and the second flexible member 818B are separated and independently movable relative to each other (and/or the collection member 812).

In Figures 8A-8C, the second adhesive 826B includes a first arm 881A and a second arm 881B laterally extending from a center portion 883. Specifically, the first arm 881A and the second arm 881B laterally extend from the center portion 883 such that the second adhesive 826B and the second attachment member 896B form a generally T-shaped structure. In this arrangement, when the second attachment member 896B is secured to the user via the adhesive 826B, the center portion 883 can be located at a middle area of the user's pelvic area and/or abdomen so that the first arm 881A and the second arm 881B extend to areas adjacent to the middle area of the user's pelvic area and/or abdomen. As noted above, this can help to reduce (or minimize) contact between the second adhesive 826B and the user's hair, and/or improve stability of the urine collection device 800 secured to the user.

In one implementation, a surface of the second adhesive 826B which faces the user can have an active adhesive portion that is configured to adhere to the user. A portion of the second adhesive 826B which faces the user can have an inactive adhesive portion that does not adhere to the user, such as the center portion 883 between the first arm 881A and the second arm 881B. This can further assist in reducing (or minimizing) an extent to which the second adhesive 826B adheres to the hair of the user at the middle area of the user's pelvic area and/or abdomen. In another implementation, the active adhesive portion of the second adhesive 826B can include at least a portion (or an entirety) of the first arm 1681A, the second arm 881B, and/or the center portion 883 of the second adhesive 826B, such that the second adhesive 826B can adhere to the user at the first arm 881A, the second arm 881B, and/or the center portion 883 of the second adhesive 826B.

As shown in Figures 8C-8D, the proximal end 832 of the collection member 812 includes an opening 836 that provides access to an internal cavity 838, which has a first chamber 838A in communication (i.e., fluid communication) with a second chamber 838B. Like the urine collection devices 100, 200, 400, 600, 700 described above, the urine collection device 800 includes an inner wall 890C that separates the first chamber 838A from the second chamber 838B. The urine collection device 800 includes an inner wall 890C separating the first chamber 838A from the second chamber 838B and the inner wall 890C includes a plurality of perforations 892 through which the first chamber 838A communicates with the second chamber 838B.

Additionally, as shown in Figures 8A and 8D, the urine collection device 800 includes, in the first chamber 838A, a permeable layer 890D and a wicking layer 890E. The wicking layer 890E is adjacent to the inner wall 890C and the permeable layer 890D is adjacent to the wicking layer 890E (i.e., the wicking layer 890E is between the permeable layer 890D and the inner wall 890C in the first chamber 838A). In this arrangement, when a penis is inserted by a user through the opening 836, the penis contacts the permeable layer 890D in the first chamber 838A while the wicking layer 890E and the inner wall 890C are below the permeable layer 890D.

In general, the permeable layer 890D can help to maintain the penis on a relatively dry surface, which in turn improves sanitary conditions, reduces the risk of infection, and/or improves user comfort. Within examples, the permeable layer 890D can be made from a porous material, which may be hydrophilic or hydrophobic. As such, the permeable layer 890D can direct urine received in the first chamber 838A toward the second chamber 838B. In one implementation, due to the hydrophobicity of the permeable layer 890D, the permeable layer 890D can repel urine and/or moisture in a direction away from the penis toward the wicking layer 890E.

As one example, the permeable layer 890D can be made from a polymer spunbond material. Additionally, in an example, the permeable layer 890D can be formed by treating a material (e.g., a natural fiber material and/or a synthetic fiber material) with a surfactant, which lowers a surface tension of fluids (e.g., the urine and/or sweat) and forms a moisture transfer channel through the permeable layer 890D to facilitate transferring the fluids or moisture from a side of the permeable layer 890D that engages the penis (e.g., in the first chamber 838A) to a side of the permeable layer 890D that faces the wicking layer 890E (i.e., in a direction from the top wall 890A toward the bottom wall 890B).

The wicking layer 890E can assist in pulling the urine, moisture, or sweat through the permeable layer 890D toward the inner wall 890C and the second chamber 838B. For example, the wicking layer 890E can be made from a material that is configured to provide for capillary action to move the urine, moisture, or sweat from a side of the wicking layer 890E facing the permeable layer 890D to a side of the wicking layer 890E facing the inner wall 890C (i.e., in the direction from the top wall 890A toward the bottom wall 890B). In particular, for example, the wicking layer 890E can be configured to provide for transverse wicking of the urine, moisture, and/or sweat from the permeable layer 890D to the inner wall 890C (and, thus, the second chamber 838B). As one example, the wicking layer 890E can be made from a mechanically absorbent polyester mesh material.

As shown in Figures 8A and 8D, a spacer 894 is in the second chamber 838B. Additionally, the urine collection device 800 includes an outlet 830 for egressing urine from the internal cavity 838 of the collection member 812. As described above, the spacer 894 can, for example, help to separate the bottom wall 890B from the inner wall 890C. This can help to inhibit (or prevent) an occlusion of the second chamber 838B, which may negatively impact egress of the urine from the urine collection device 800. Additionally, for example, the spacer 894 can help to inhibit (or prevent) an occurrence of a vacuum lock condition due to the vacuum pressure applied by a vacuum device.

As shown in Figures 8A-8B, the outlet 830 can include a port 878 coupled to the spacer 894 by a tube 879 extending through a seam 899 at the distal end 834 of the collection member 812 (e.g., at a distalmost point of the collection member 812). Additionally, the port 878 can include a tapered end portion 878A, which can help to more universally couple the port 878 to a plurality of differently sized drain tubes. In some examples, the port 878 can be rotatable relative to the collection member 812. In other examples, the port 878 can be rotationally fixed relative to the collection member 812.

The tube 879 can assist in spacing a point of connection between the urine collection device 800 and a drain tube farther away from the patient. This can help to improve handling by medical practitioners as the tube 879 can provide for greater flexibility and range of movement of the port 878 while coupling the port 878 to the drain tube and/or decoupling the port 878 from the drain tube. Additionally, the tube 879 can help to reduce a risk that a medical practitioner will inadvertently decouple the urine collection device 800 from the user when the medical practitioner manipulates the location and/or orientation of the port 878 to couple the port 878 with the drain tube.

As shown in Figure 8A, the tube 879 can extend within the spacer 894. This can help to reduce (or prevent) a risk of occlusion due to vacuum suction and adhesion of the inner wall 890C and the bottom wall 890B. As an example, the tube 879 can be coupled to the spacer 894 by bonding via solvents, adhesives, welding, and/or snap-fit.

In this arrangement, the urine can be initially received in the first chamber 838A of the internal cavity 838. The urine may initially contact the permeable layer 890D, which supports the penis in the first chamber 838A. The urine can pass through the permeable layer 890D to the wicking layer 890E, which can provide capillary action to move the urine from the permeable layer 890D to the inner wall 890C. The urine can then pass through the perforations 892 in the inner wall 890C to the second chamber 838B. In the second chamber 838B, the urine can flow distally and egress from the collection member 812 at the outlet 830. As described above, this flow of the urine through the collection member 812 can be assisted by the hydrophobicity of the permeable layer 890D, the capillary action of the wicking layer 890E, gravity, and/or a vacuum pressure applied by a vacuum device at the outlet 830 of the collection member 812.

Although the collection member 812 includes the inner wall 890C, the permeable layer 890D, and the wicking layer 890E in Figures 8A-8D, the collection member 812 can include omit one or two of these layers in other examples. For instance, as one example, the collection member 812 can include the permeable layer 890D and the wicking layer 890E but omit the inner wall 890C.

Additionally, as shown in Figures 8A-8D, the urine collection device 800 can include a label 875. The label 875 can provide for recording operation information (e.g., a time of securement of the urine collection device 800 to the patient), which can help medical personnel more readily identify information related to patient care. In Figures 8A-8C, the label 875 is on the second arm 881B, which can provide for easy access on an outer side of the urine collection device 800 when the urine collection device 800 is secured to a user. However, the label 875 can be at additional or alternative locations on the urine collection device 800 in other examples.

As described above, the collection members are suitable to direct urine to an outlet of the collection member, and the outlet is suitable to egress the urine from the collection member. In some examples, however, the collection member can be configured to retain a volume of urine in instances in which a drain tube is occluded and/or closed (e.g., by a valve). In one implementation, the collection member can be suitable to contain at least approximately 400 milliliters (ml) of urine.

Additionally, as described above, the urine collection device 100, 200, 400, 600, 700, 800 can include an inner wall 190C, 490C, 690C, 790C, 890C that includes a plurality of perforations 192, 292, 492, 692, 792, 892. In the examples shown in Figures 1A-4 and 6-7D, the perforations 192, 292, 492, 692, 792, 892 are all approximately the same size as each other. However, in another example, the perforations 192, 292, 492, 692, 792, 892 can have different sizes.

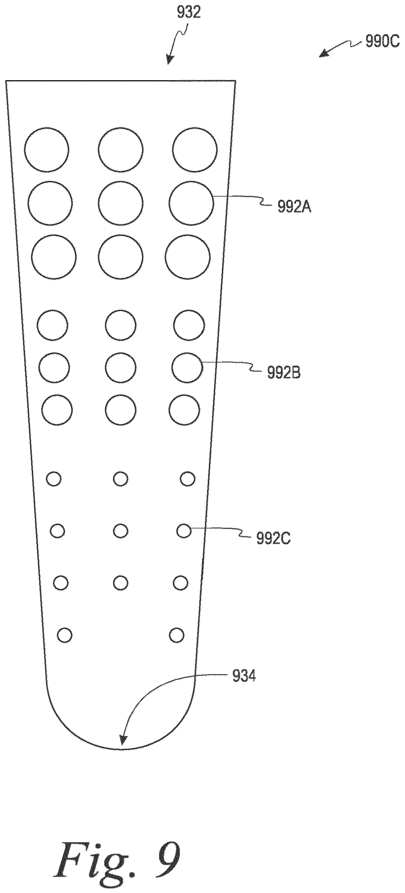

As one example, Figure 9 depicts an inner wall 990C having a plurality of perforations 992A-992C of a plurality of different sizes and which can be used with any of the urine collection devices 100, 200, 400, 600, 700, 800 described herein. In Figure 9, the perforations 992A-992C include a plurality of first perforations 992A each having a first size, a plurality of second perforations 992B each having a second size, and a plurality of third perforations 992C each having a third size. The first perforations 992A are proximal of the second perforations 992B, which are proximal of the third perforations 992C. In this example, the first size of the first perforations 992A is larger than the second size of the second perforations 992B, which is larger than the third size of the third perforations 992C.

Thus, in Figure 9, the perforations 992A-992C decrease in size along a direction from the proximal end 932 to a distal end 934. This can help to improve suction when the urine collection device 100, 200, 400, 600, 700, 800 is used with a vacuum device and/or more rapidly transfer urine from the first chamber 138A, 238A, 838A to the second chamber 138B, 238B, 838B.

In Figure 9, the perforations 992A-992C include three different sizes. However, in another example, the perforations 992A-992C can include two different sizes or more than three different sizes. For instance, in one implementation, the perforations 992A-992C can continuously and progressively decrease in size along the direction from the proximal end 932 to the distal end 934. Additionally, in other examples, the perforations 992A-992C can increase in size along the direction from the proximal end 932 to the distal end 934 (i.e., the first perforations 992A can be smaller than the second perforations 992B and the second perforations 992B can be smaller than the third perforations 992C). In yet another example, the perforations 992A-992C can be arranged such that relatively large sized perforations 992A are intermixed with relatively small size perforations 992B, 992C along the direction from the proximal end 932 to the distal end 934.

In examples described above, the inner wall 190C, 490C, 690C, 790C, 890C, 990C can be made from a liquid impermeable material (e.g., a plastic material) such that the urine can flow from the first chamber 138A, 238A, 838A to the second chamber 138B, 238B, 838B through the perforations 192, 292, 492, 692, 792, 892, 992A-992C. In such examples, the perforations 192, 292, 492, 692, 792, 892, 992A-992C can thus define fluid passageways through the inner wall 190C from the first chamber 138A, 238A, 838A to the second chamber 138B, 238B, 838B.

In another example, the inner wall 190C, 490C, 690C, 790C, 890C, 990C can be combined with the permeable layer 890D to define such fluid passageways from the first chamber 138A, 238A, 838A to the second chamber 138B, 238B, 838B. For instance, the permeable layer 890D can be configured to have (i) a first zone that inhibits the transfer of urine from a side of the permeable layer 890D that faces the first chamber 838A to a side of the permeable layer 890D that faces the second chamber 838B, and (ii) and a second zone that allows the transfer of urine from the side of the permeable layer 890D that faces the first chamber 838A to the side of the permeable layer 890D that faces the second chamber 838B. In one implementation, the first zone and the second zones can be formed by applying a surface treatment (e.g., a coating) to the permeable layer 890D at the first zone, but not the second zones. The surface treatment can include, for instance, a hydrophobic coating and/or a liquid repellant coating (e.g., a fluoropolymer).

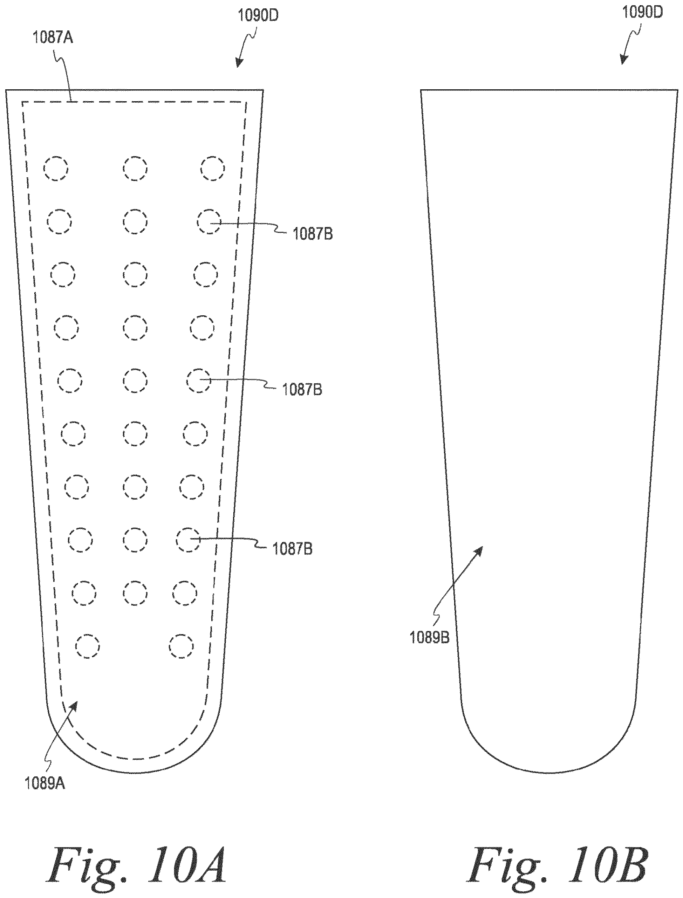

The first zone and the second zones can be arranged in a pattern on the permeable layer 890D in manner similar to the patterns illustrated and described for the perforations 192, 292, 492, 692, 792, 892, 992A-992C on the inner walls 190C, 490C, 690C, 790C, 890C, 990C. As one example, Figures 10A-10B depict a permeable layer 1090D that includes a first zone 1087A and a plurality of second zones 1087B. In particular, Figure 10A depicts a side 1089A of the permeable layer 1090D that can face the first chamber 138A, 238A, 838A of the urine collection devices 100, 200, 400, 600, 700, 800 and Figure 10B depicts a side 1089B of the permeable layer 1090D that can face the second chamber 138B, 238B, 838B of the urine collection devices 100, 200, 400, 600, 700, 800. As described above, the first zone 1087A can include the surface treatment that inhibits the transfer of the urine from the first side 1089A of the permeable layer 1090D facing the first chamber to the second side 1089B of the permeable layer 1090D facing the second chamber.

Also, as described above, the second zones 1087B omit the surface treatment. In this arrangement, when the urine contacts the side 1089A facing the first chamber, the urine will flow over and around the first zone 1087A to the second zones 1087B and, at the second zones 1087B, the urine will transfer through the permeable layer 1090D from the first side 1089A facing the first chamber to the second side 1089B facing the second chamber.

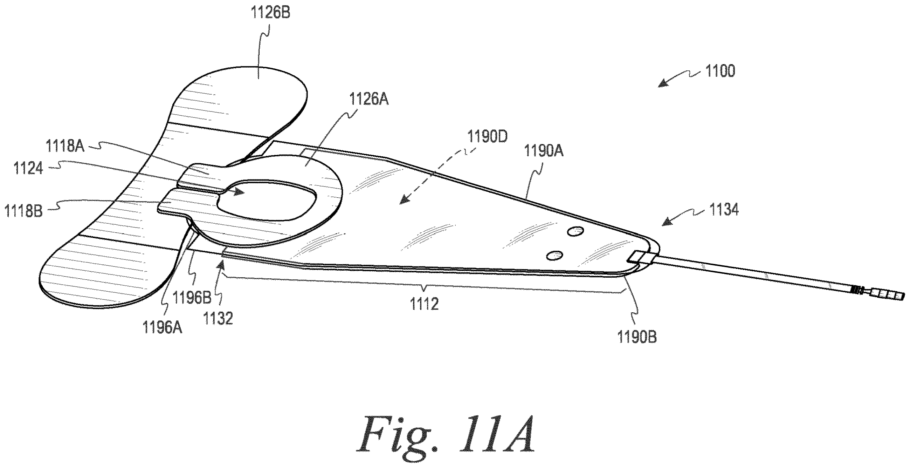

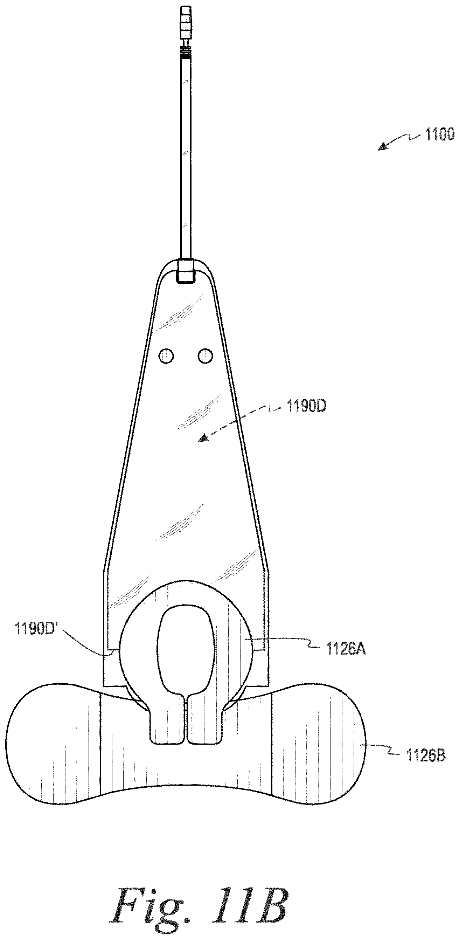

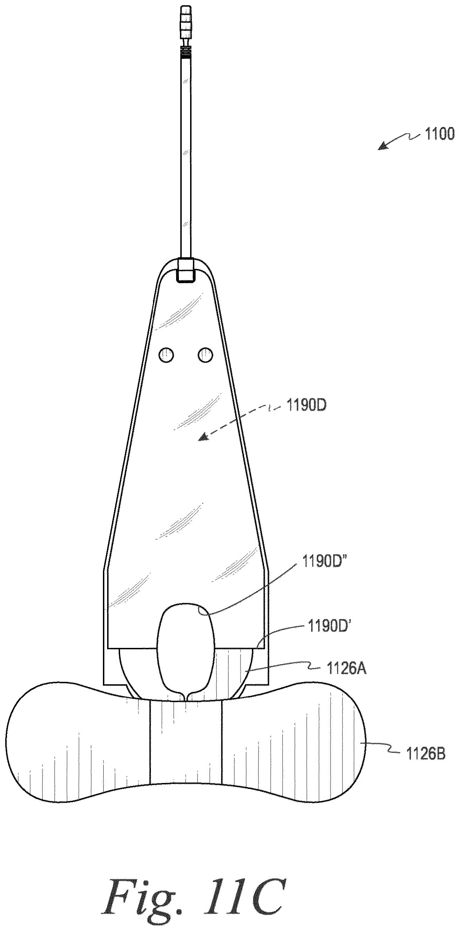

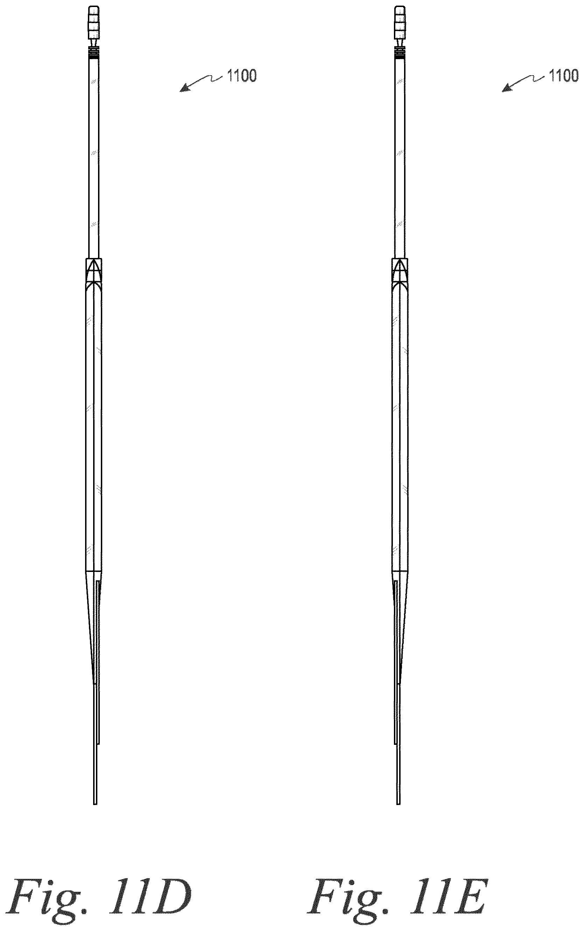

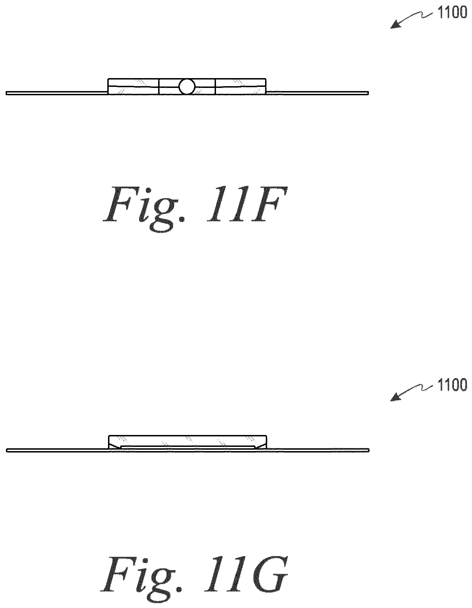

Referring now to Figures 11A-11E, a urine collection device 1100 is depicted according to another example embodiment. Figure 11A depicts a perspective view of the urine collection device 1100, Figure 11B depicts a top view of the urine collection device 1100, Figure 11C depicts a bottom view of the urine collection device 1100, Figure 11D depicts a first side view of the urine collection device 1100, Figure 11E depicts a second side view of the urine collection device, Figure 11F depicts a third side view of the urine collection device, and Figure 11G depicts a fourth side view of the urine collection device. The urine collection device 1100 is substantially similar to the urine collection devices 100, 200, 400, 600, 700, 800 described above.

For example, the urine collection device 1100 includes a collection member 1112 extending from a proximal end 1132 to a distal end 1134. A first attachment member 1196A extends from a bottom wall 1190B of the collection member 1112 at the proximal end 1132, and a second attachment member 1196B extends from a top wall 1190A of the collection member 1112 at the proximal end 1132. The first attachment member 1196A also includes a first adhesive 1126A, and the second attachment member 1196B includes a second adhesive 1126B to assist in securing the urine collection device 1100 to a user as described above.

The first attachment member 1196A includes a first flexible member 1118A and a second flexible member 1118B. The first flexible member 1118A and the second flexible member 1118B of the first attachment member 1196A define an aperture 1124 in the first attachment member 1196A. In Figures 11A-11C, the first attachment member 1196A and/or the first adhesive 1126A define a shape of the aperture 1124. For instance, in Figures 11A-11C, the first attachment member 1196A and/or the first adhesive 1126A define a generally oblong and/or a substantially elliptical shape of the aperture 1124. Within examples, the generally oblong and/or a substantially elliptical shape of aperture 1124 can assist in improving securing the first attachment member 1196A to the user in relatively close proximity to the penis of the user.

Additionally, the urine collection device 1100 includes an inner wall (not shown), a permeable layer 1190D, and/or a wicking layer (not shown) between a first chamber (not shown) and a second chamber (not shown) as described above with respect to Figures 8A-8D. However, the permeable layer 1190D in Figures 11A-1C extends more proximally than the permeable layer 890D in Figures 8A-8D. Specifically, in Figures 11A-11C, the permeable layer 1190D can be arranged in the urine collection device 1100 such that a proximal-most portion 1190D' of the permeable layer 1190D overlaps with at least a portion of the first adhesive 1126A. For instance, as shown in Figure 11C, the proximal-most portion 1190D' of the permeable layer 1190D can include a notch 1190D" having a shape that corresponds to a shape of an inner edge of the first adhesive 1126A. By arranging the permeable layer 1190D with the proximal-most portion 1190D' overlapping with at least a portion of the first adhesive 1126A, the permeable layer 1190D can assist in mitigating (or preventing) leakage of urine from the urine collection device 1100 when a user is turned (e.g., relative to a hospital bed).

In another example, the permeable layer 1190D can entirely overlap and/or extend proximally of the first adhesive 1126A. This can further assist in mitigating (or preventing) or preventing leakage. Additionally, this can help to improve patient comfort by providing covering a greater surface area of the urine collection device with the permeable layer 1190D, which can be relatively soft and gentle on the skin of the patient. Examples in which a permeable layer overlaps an entirety of the first adhesive are shown in Figures 16-17B.

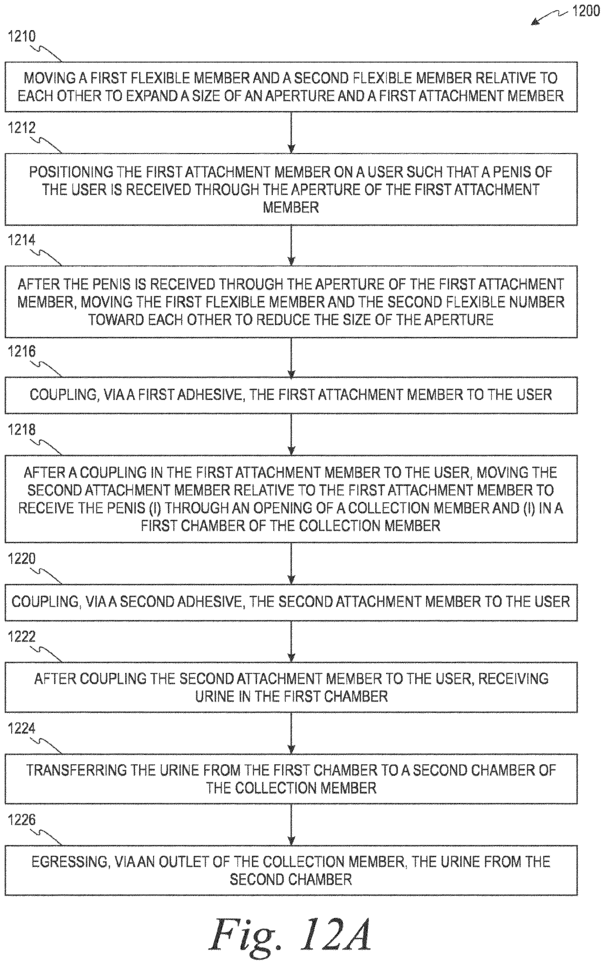

Referring now to Figure 12A, a flowchart for a process 1200 for collecting urine is illustrated according to an example embodiment. As shown in Figure 12A, at block 1210, the process 1200 includes moving a first flexible member and a second flexible member relative to each other to expand a size of an aperture in the first attachment member. At block 1212, the process 1200 includes positioning the first attachment member on a user such that a penis of the user is received through the aperture. After the penis is received through the aperture of the first attachment member at block 1212, the process 1200 includes moving the first flexible member and the second flexible member toward each other to reduce the size of the aperture at block 1214. At block 1216, the process 1200 includes coupling, via a first adhesive, the first attachment member to the user. After coupling the first attachment member to the user at block 1216, the process 1200 includes moving the second attachment member relative to the first attachment member to receive the penis (i) through an opening of a collection member and (ii) in a first chamber of the collection member at block 1218. At block 1220, the process 1200 can include coupling, via a second adhesive, the second attachment member to the user.

After coupling the second attachment member to the user at block 1220, the process 1200 can include receiving urine in the first chamber at block 1222. At block 1224, the process 1200 can include transferring the urine from the first chamber to a second chamber of the collection member. At block 1226, the process 1200 can include egressing, via an outlet of the collection member, the urine from the second chamber.

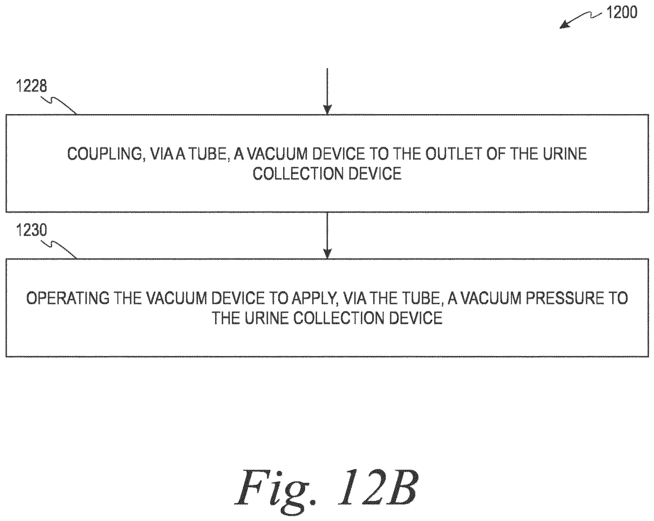

Figure 12B depicts additional aspects of the process 1200 according to further examples. As shown in Figure 12B, the process 1200 can also include coupling, via a tube, a vacuum device to the outlet of the urine collection device at block 1228. At block 1230, the process 1200 can include operating the vacuum device to apply, via the tube, a vacuum pressure to the urine collection device.

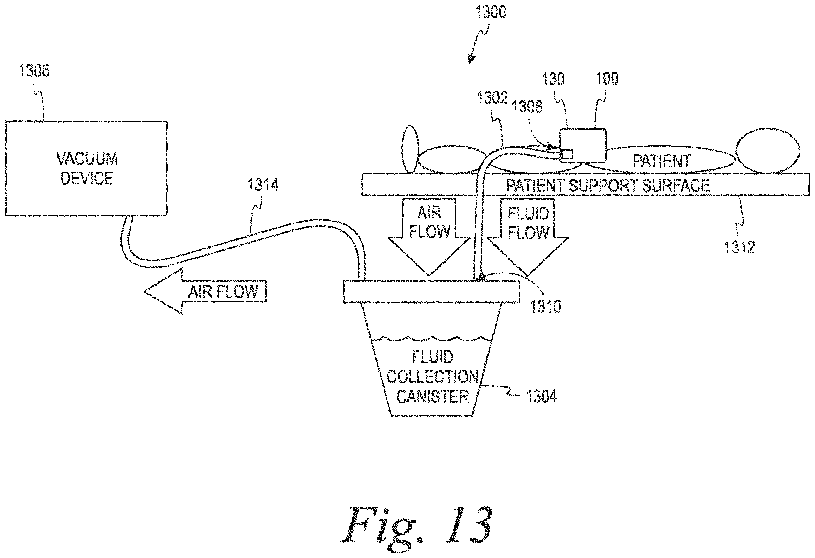

Referring now to Figure 13, a simplified diagram of a system 1300 for collecting urine is depicted according to an example embodiment. As shown in Figure 13, the system 1300 includes the urine collection device 100 described above, although the system 1300 can operate with any of the urine collection devices 100, 200, 400, 600, 700, 800, 1100 described herein. Additionally, the system 1300 includes a drain tube 1302, a waste collection reservoir 1304, and a vacuum device 1306.

A first end 1308 of the drain tube 1302 is coupled to the outlet 130 of the collection member 112. For example, the drain tube 1302 can include a thread, a Luer lock, and/or other feature for coupling the drain tube 1302 to the outlet 130. Within examples, the drain tube 1302 can be a flexible material to facilitate directing the drain tube away from the user's body. It can be beneficial to direct the drain tube 1302 away from the user's body (e.g., off the side of a bed) to reduce (or prevent) the drain tube 1302 from accidental pulling and leakage resulting from such pulling.

The waste collection reservoir 1304 is coupled to a second end 1310 of the drain tube 1302 to receive the urine from the drain tube 1302. In one example, the waste collection reservoir 1304 can be a leg bag, a drainage bag, or other container. In another example, the waste collection reservoir 1304 can include a hanger and/or another structure for coupling the waste collection reservoir 1304 to a patient support surface 1312 (e.g., a bed and/or a wheelchair) used by the patient.

In some examples, the waste collection reservoir 1304 can be a sealed container. This can, for example, reduce (or minimize) a risk of spillage and/or contamination. In some examples, the waste collection reservoir 1304 can be disposable. In other examples, the waste collection reservoir 1304 can be reusable. For instance, the waste collection reservoir 1304 can be configured to be sterilized after a use and reused.

The vacuum device 1306 can apply a vacuum pressure to the drain tube 1302 to assist in directing the urine from the outlet 130 to the waste collection reservoir 1304. For instance, the vacuum device 1306 can include an air pump or other vacuum source, which is coupled to the waste collection reservoir 1304 by an air tube 1314. In one example, the air tube 1314 can also be made of a flexible material.

In some examples, the vacuum device 1306 can be a wall vacuum integrated into a room of a medical facility. In other examples, the vacuum device 1306 can be integrated with the patient support surface 1312. For instance, the vacuum device 1306 can be integrated with a bed in a medical facility. The vacuum device 1306 can then be operated to apply the vacuum pressure at the outlet 130 (e.g., via the air tube 1314, the waste collection reservoir 1304, and the drain tube 1302).

Within some examples, the system 1300 can also include an occlusion clip for selectively controlling the flow of urine in the drain tube 1302. For instance, the occlusion clip can provide for stopping the flow of urine in the drain tube 1302 to facilitate changing and/or emptying the waste collection reservoir 1304.

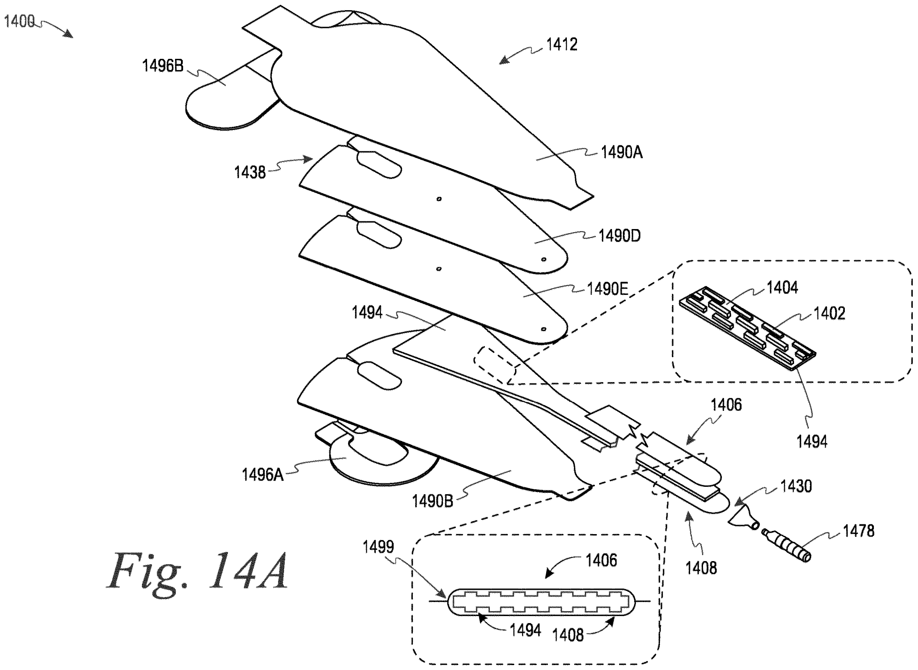

Referring now to Figure 14A, a urine collection device 1400 is described according to another example. The urine collection device 1400 of Figure 14A is substantially similar to the urine collection devices 100, 200, 400, 600, 700, 800, 1100 described above. For instance, the urine collection device 1400 includes an internal cavity 1438 to collect urine discharged from a body of a user. The urine collection device 1400 further includes an outlet 1430 in fluid communication with the internal cavity 1438. The urine collection device 1400 further includes a spacer 1494 positioned between the internal cavity 1438 and the outlet 1430. The spacer 1494 comprises a pliable film including a plurality of protrusions 1402 and a plurality of through-holes 1404, and the spacer 1494 is in fluid communication with the internal cavity 1438 and the outlet 1430 such that urine is directed from the internal cavity 1438, through the spacer 1494, and to the outlet 1430.

The arrangement described above helps to improve the fluid flow across the urinary management devices (and similar suction devices such as fecal management system) by incorporating a flat profile suction pathway containing multiple flow channels defined by the plurality of protrusions 1402 and a plurality of through-holes 1404 of the spacer 1494. Some existing urine management devices utilize polyurethane tubing to move fluids away from fragile and sensitive skin using an external vacuum pump. The male urinary management device contains a patient contacting component that is connected to the polyurethane tube which moves fluid away from the body into an external canister under vacuum. Since the patient contacting component is constructed using a relatively thin film (~0.003 inches) of polyurethane whereas the tube walls are significantly thicker (0.125 inches, and has more tensile strength), the film component may collapse under the application of vacuum creating a potential blockage for fluid flow.

Further, during normal use conditions, the urine collection device may get twisted at the interface of tubing and patient contacting film layer preventing fluid flow. In addition, for both male and female urinary management devices, while clinicians take significant care to ensure that the polyurethane tubing is not blocked or tangled, external objects (such as movement of medication transport trolley or clinician stepping on to the tube) or patient's own weight may block the tubing. As such, in one example, the urine collection device 1400 described above may be configured to collect urine from a female anatomy. In another embodiment, the urine collection device 1400 described above may be configured to collect urine from a male anatomy.

To overcome these potential issues, the urine collection device 1400 described above provides a design of a flat profile, pliable flow channel with embossed pattern (i.e., island of raised areas) (e.g., the spacer 1494) that may transmit vacuum using multiple channels (instead of one single channel like existing tubing with circular cross-section). Further, the configurations of the urine collection device 1400 described above prevents thin film collapsing under vacuum, provides better resistance to twisting and still works when twisted, and has improved ability to move fluid even when physically blocked by external weight. In addition, the urine collection device 1400 described above may enable replacement of polyester foam with polyurethane film, which provides potential cost saving opportunities.

As shown in Figure 14A, the urine collection device 1400 of Figure 14A is substantially similar to the urine collection devices 100, 200, 400, 600, 700, 800, 1100 described above. In particular, the urine collection device 1400 may include a collection member 1412 including a top wall 1490A, a permeable layer 1490D, a wicking layer 1490E, the spacer 1494, a bottom wall 1490B, a first attachment member 1496A extending from the bottom wall 1490B, and a second attachment member 1496B extending from the top wall 1490A.

In one example, the outlet 1430 comprises a port 1478 configured to couple to a drain tube of a vacuum device (e.g., drain tube 1302 of vacuum device 1306). In one example, the port 1478 is rotatable with respect to the collection member 1412. Such an arrangement may help to prevent twisting of the collection member 1412 with respect to the tubing.

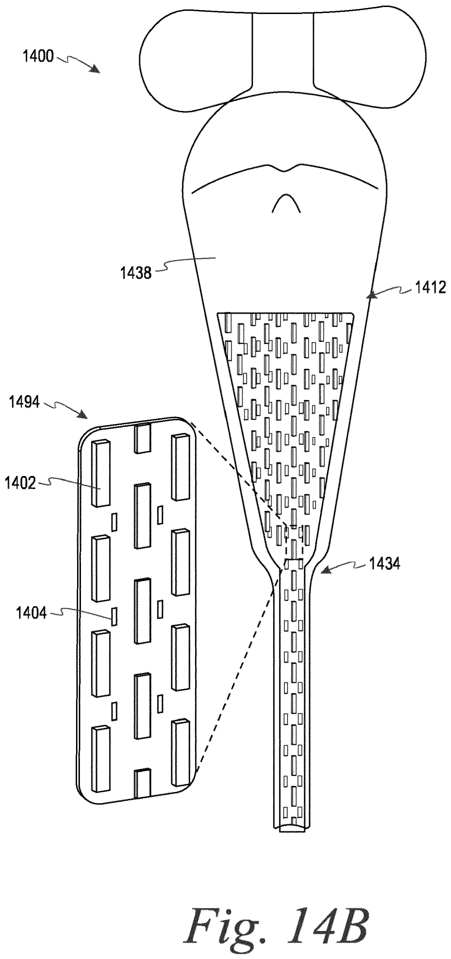

In one example, as shown in Figure 14B, the spacer 1494 extends distally from a distalmost point 1434 of the collection member 1412. In such an example, as shown in the side cross-sectional view of Figure 14A, the urine collection device 1400 may further comprise a first sheet of material 1406 coupled to a second sheet of material 1408 to thereby surround the spacer 1494. As shown in Figure 14B, the outlet 1430 is positioned the distalmost point 1434 of the spacer at a seam 1499 between the first sheet of material 1406 and the second sheet of material 1408.

The spacer 1494 may take a variety of forms. In one example, the spacer 1494 comprises one or more of polyurethane, polyamide, polypropylene, polyethylene, polyvinyl chloride, ethylene vinyl acetate copolymer, polyether block amide polymers, silicone, or polyurethane thermoplastic. In another example, a thickness of the spacer 1494 ranges from about 30 µm to about 5 mm. In another example, the spacer 1494 is coated with one or more antimicrobial agents. The one or more antimicrobial agents may be selected from the group consisting of silver, acetic acid, polyhexamethylene biguanide, or combinations thereof.

The plurality of protrusions 1402 of the spacer 1494 may take a variety of forms. In another example, air is trapped in the plurality of protrusions 1402 of the spacer 1494. In another example, the plurality of protrusions 1402 in the spacer 1494 comprise one or more of circular, triangular, rectangular, or polygonal geometry. In another example, a height of the plurality of protrusions 1402 range from about 500 µm to about 2 cm. In one example, the height of the plurality of protrusions 1402 are uniform. In another example, the height of the plurality of protrusions 1402 are non-uniform. In one example, a lineal dimension of the plurality of protrusions range from about 1 mm to about 2 cm. In one example, as shown in Figure 14A, the plurality of protrusions 1402 are positioned on each side of the spacer 1494. In another example, the plurality of protrusions 1402 are encompassed between two flat film structures (e.g., the first sheet of material 1406 and the second sheet of material 1408) such that flow channels are present within a bilayer of the pliable film itself. In another example, each of the plurality of through-holes 1404 have a length ranging from about 1 mm to about 5 mm.

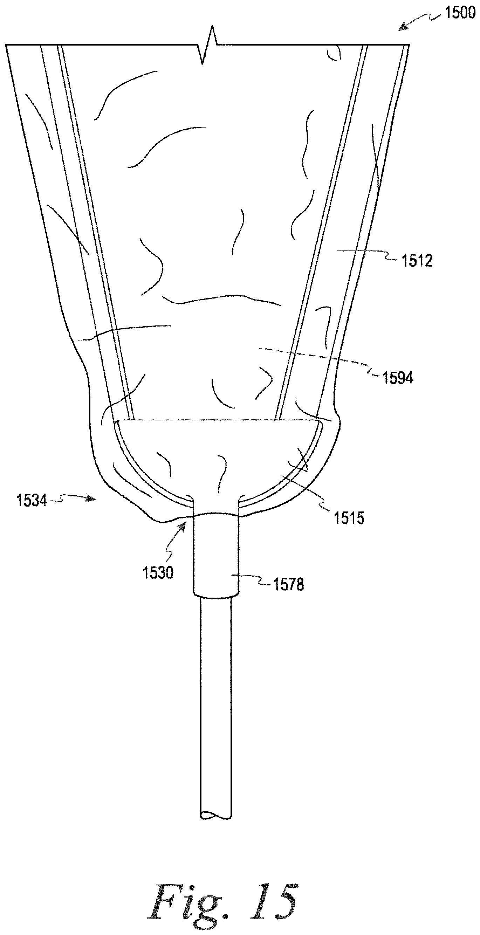

Referring now to Figure 15, a distal portion of a urine collection device 1500 is shown according to an example. The urine collection device 1500 can be substantially similar or identical to any of the urine collection devices 100, 200, 400, 600, 700, 800, 1100 described above. However, an outlet 1530 of the urine collection device 1500 shown in Figure 15 includes a port support structure 1515 at a distal end 1534 of a collection member 1512.

As shown in Figure 15, the port support structure 1515 can be in the internal cavity 1538 of the collection member 1512. Additionally, the port support structure 1515 can extend across a width of the collection member 1512 at the distal end 1534 (e.g., the port support structure 1515 can extend entirely across the width between a first lateral side and a second lateral side of the collection member 1512). In this arrangement, the relatively wide shape and size of the port support structure 1515 can help to mitigate (or prevent) twisting of the collection member 1512 at the distal end 1534.

To further assist in mitigating twisting, the port support structure 1515 can be formed from a material having a rigidity that is greater than a rigidity of a material of the collection member 1512. Additionally, for example, the port support structure 1515 can define a recess at a proximal side of the port support structure 1515, and the spacer 1594 can be received in the recess of the port support structure 1515. This can help to direct urine from the spacer 1594 into the port support structure 1515.

As shown in Figure 15, the port support structure 1515 and a port 1578 can be integrally formed as a single, monolithic structure. However, in other examples, the port 1578 can be separate component that is coupled to the port support structure 1515. Also, in Figure 15, the port 1578 is rotationally fixed relative to the port support structure 1515 and the collection member 1512. However, in other examples, the port 1578 can be rotatable relative to the port support structure 1515 and the collection member 1512.

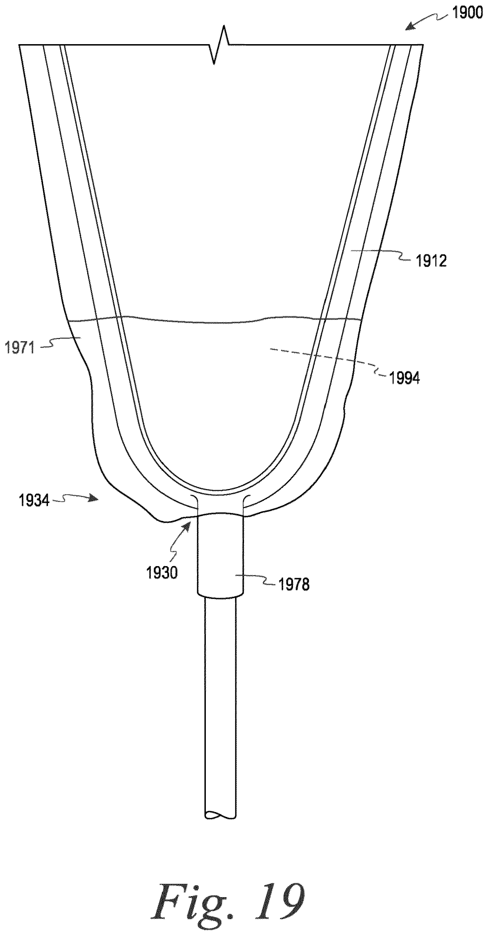

Referring now to Figure 19, a distal portion of a urine collection device 1900 is shown according to an example. The urine collection device 1900 can be substantially similar or identical to any of the urine collection devices 100, 200, 400, 600, 700, 800, 1100, 1500 described above. However, a distal portion of a collection member 1912 can include a reinforcement member 1971 on at least one of the top wall or a bottom wall (e.g., the top wall 190A or the bottom wall 190B in Figure 1A). As an example, the reinforcement member 1971 can be additional layer of material such as, for instance, an additional layer of the same material from which the top wall and/or the bottom wall are formed.

As shown in Figure 19, the reinforcement member 1912 can extend in a proximal direction from a distal end 1934 of the collection member 1912. Additionally, the reinforcement member 1971 can extend across a width of the collection member 1912 at the distal end 1934 (e.g., the reinforcement member 1971 can extend entirely across the width between a first lateral side and a second lateral side of the collection member 1912). In this arrangement, the reinforcement member 1971 can enhance a rigidity of the distal portion of the collection member 1912 (e.g., at an outlet 1930 and/or a port 1978) can help to mitigate (or prevent) twisting of the collection member 1912 at the distal end 1934.

Referring now to Figure 16, a urine collection device 1600 is shown according to another example. The urine collection device 1600 can be substantially similar or identical to any of the urine collection devices 100, 200, 400, 600, 700, 800, 1100, 1400, 1500, 1900 described above. However, a collection member 1612 of the urine collection device 1600 can include a plurality of seams 1617 on opposing sides of an opening 1636 at a proximal end 1632 of the collection member 1612, which can help to mitigate leakage of urine out the opening 1636 at the proximal end 1632.

As shown in Figure 16, each of the seams 1617 can extend inwardly from a respective lateral edge 1619 of the collection member 1612 towards a middle portion of the collection member 1612. The opening 1636 for receiving the penis is at the middle portion between the seams 1617 (e.g., the opening 1636 separates the seams 1617). In this arrangement, the seams 1617 can assist in arranging the proximal end 1632 with the opening 1636 extending across less than an entire width of the collection member 1612, thereby reducing a size of the opening 1636 relative to some implementations that omit the seams 1617.

As described above, the collection member 1612 can include a first sheet of material and a second sheet of material (e.g., the top sheet, the bottom sheet, and/or the inner layer described above). As examples, the seams 1617 can be formed by coupling the first sheet of material and the second sheet of material to each other at the seams 1617 (e.g., via RF heat sealing and/or RF welding).

In Figure 16, the opening 1636 has a fixed size defined by the distance between the seams 1617. In other examples, the opening can have an adjustable size.

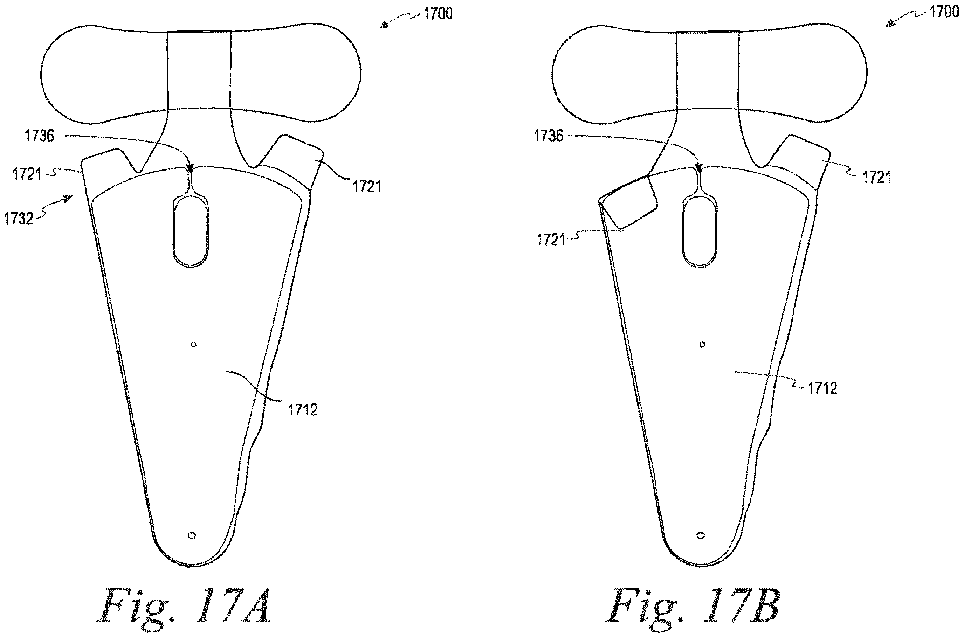

As an example, Figures 17A-17B depict a urine collection device 1700 in which a size of an opening 1736 is adjustable, according to an example. The urine collection device 1700 can be substantially similar or identical to any of the urine collection devices 100, 200, 400, 600, 700, 800, 1100, 1400, 1500 described above. However, a collection member 1712 of the urine collection device 1700 includes a plurality of tabs 1721 that extend outwardly from the proximal end 1732.

As shown in Figure 17A, each tab 1721 is also positioned at a respective one of the lateral sides 1719. Additionally, each tab 1721 is movable relative to the collection member 1712, and each tab 1721 is configured to couple to an outer surface of the collection member 1712 to cover at a respective portion of the opening 1736 at the proximal end 1732. For example, each tab 1721 can include an adhesive that can couple the tab 1721 to the outer surface of the collection member 1712.

In this arrangement, the opening 1736 can have a first size when the tabs 1721 are decoupled from the collection member 1712, the opening can have a second size when the tabs 1721 extend across respective portions of the opening 1736 and couple to the collection member 1712, and the first size can be greater than the second size. Figure 17A illustrates the urine collection device 1700 with the tabs 1721 decoupled from the collection member 1712, and Figure 17B illustrates the urine collection device 1700 after one of the tabs 1721 is coupled to the outer surface of the collection member 1712. In use, the other of the tabs 1721 can also be coupled to the outer surface of the collection member 1712. As shown in Figure 17B, the tab 1721 that is coupled to the collection member 1712 covers a portion of the opening 1736, which reduces the effective size of the opening 1736 relative to the opening 1736 shown in Figure 17A.

In one implementation, the collection member includes a first sheet of material coupled to a second sheet of material, as described above. The tabs 1721 can extend from the first sheet of material at the proximal end 1732, and each tab 1721 can extend over the portion of the opening 1736 and couple to the second sheet of material. For instance, in Figures 17A-17B, the tabs 1721 extend from the bottom wall and can couple to the top wall. However, in other examples, the tabs 1721 can extend from the top wall and couple to the bottom wall. In still other examples, one tab 1721 can extend from the top wall and the other tab 1721 can extend from the bottom wall such that the tabs 1721 extend from different sheets of material and couple to different sheets of material for the collection member 1712.

In use, after the user inserts the penis through the opening 1736, the size of the opening 1736 can be adjusted by coupling the tabs 1721 to the collection member 1712. For instance, a sacrificial liner can be removed to expose the adhesive on the tabs 1721, the tabs 1721 can be folded over the proximal end 1732 and coupled to the outer surface of the collection member 1712. This arrangement can allow for the size of the opening 1736 to be tailored to a size that is well suited for a particular patient's anatomy extending through the opening 1736, and help mitigate leakage of urine out the opening 1736 at the proximal end 1732.

The urine collection devices 100, 200, 400, 600, 700, 800, 1100, 1400, 1500, 1600, 1700, 1900 described above and shown in Figures 1A-17B are representative examples of implementations of urine collection devices, systems, and methods in accordance with aspects of the present disclosure. The features described and illustrated with respect to the urine collection devices 100, 200, 400, 600, 700, 800, 1100, 1400, 1500, 1600, 1700, 1900 can be modified and combined according to additional or alternative examples of the present disclosure.

The following are a few of the many modifications and/or combinations of features that can be implemented in accordance with the present disclosure. In some examples, any of the urine collection devices 100, 200, 400, 600, 700, 800, 1100, 1400, 1500, 1600, 1700, 1900 can include the port that is rotatable relative to the collection member, or a port that is rotationally fixed relative to the collection member. Additionally, for instance, any of the urine collection devices 100, 200, 400, 600, 700, 800, 1100, 1400, 1500, 1600, 1700, 1900 can include the baffle portions 699 shown in Figure 6, the seams 1617 shown in Figure 16, and/or tabs 1721 shown in Figures 17A-17B at the proximal end to assist in mitigating leakage, or omit such features to provide gaps for improved air flow. Also, for instance, any of the urine collection devices 100, 200, 400, 600, 700, 800, 1100, 1400, 1500, 1600, 1700, 1900 can include the port support structure 1515 shown in Figure 15, or omit the port support structure 1515. Further, for instance, any of the urine collection devices 100, 200, 400, 600, 700, 800, 1100, 1400, 1500, 1600, 1700, 1900 can include the port at the top wall, the bottom wall, and/or the distal-most point of the collection member. Additionally, for instance, any of the urine collection devices 100, 200, 400, 600, 700, 800, 1100, 1400, 1500, 1600, 1700, 1900 can include one or more layers selected from a group consisting of: the permeable layer, the wicking layer, and the inner layer with perforations. Further, in implementations that include the permeable layer and/or the wicking layer, any of the urine collection devices 100, 200, 400, 600, 700, 800, 1100, 1400, 1500, 1600, 1700, 1900 can include the permeable layer and/or the wicking layer overlapping entirely with the first adhesive, overlapping only partially with the first adhesive, or not overlapping with the first adhesive. Additionally, for instance, any of the urine collection devices 100, 200, 400, 600, 700, 800, 1100, 1400, 1500, 1600, 1700, 1900 can include the spacer 1494 shown in Figures 14A-14B. Also, for instance, any of the urine collection devices 100, 200, 400, 600, 700, 800, 1100, 1400, 1500, 1600, 1700, 1900 can include a first adhesive and/or a second adhesive that includes a stiffening member (e.g., a foam pad) or omits the stiffening member.

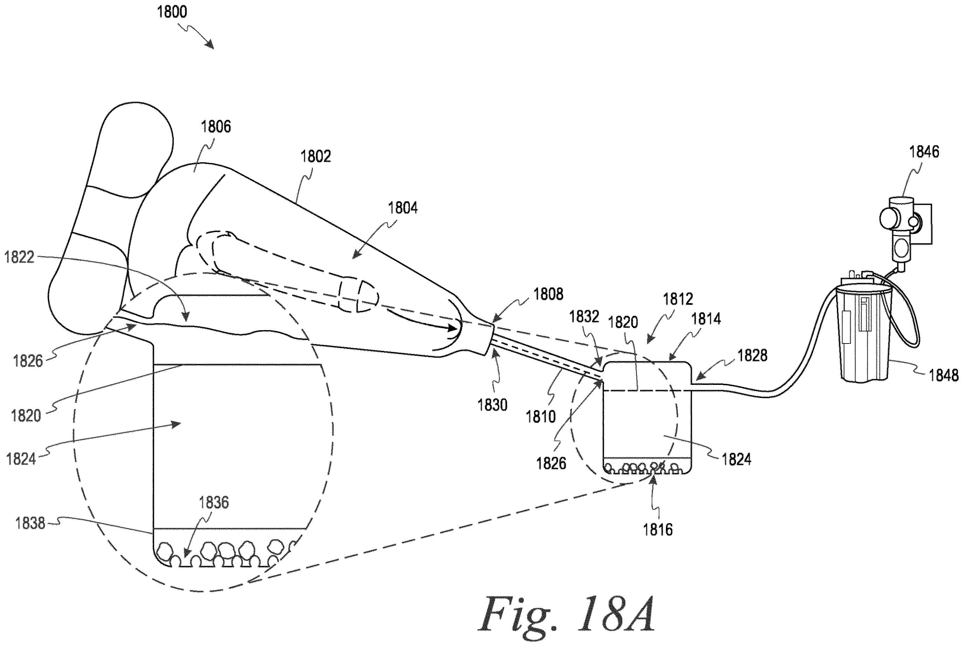

Referring now to Figure 18A, a urine collection system 1800 is shown according to an example. As shown in Figure 18A, the urine collection system 1800 includes a first fluid collection device 1802 including a first internal cavity 1804 to collect urine discharged from a body of a user. The first fluid collection device 1802 also includes a first inlet 1806 in fluid communication with the first internal cavity 1804, a first outlet 1808 in fluid communication with the first internal cavity 1804. In one example, as shown in Figures 18A-18C, the first fluid collection device 1802 is configured to collect urine from a male anatomy. For instance, the first fluid collection device 1802 can be any of the urine collection devices 100, 200, 400, 600, 700, 800, 1100, 1400, 1500, 1600, 1700, 1900 described and illustrated above with respect to Figures 1A-17B. In another example, the first fluid collection device 1802 is configured to collect urine from a female anatomy.

As shown in Figure 18A, the urine collection system 1800 also includes a tube 1810 in fluid communication with the first outlet 1808. The urine collection system 1800 also includes a second fluid collection device 1812 having a first end 1814 and a second end 1816 opposite the first end 1814. The second fluid collection device 1812 includes a second internal cavity 1818 defined between the first end 1814 and the second end 1816 of the second fluid collection device 1812. The second fluid collection device 1812 further includes a permeable membrane 1820 positioned within the second internal cavity 1818 between the first end 1814 and the second end 1816 of the second fluid collection device 1812, thereby separating the second internal cavity 1818 into a first chamber 1822 and a second chamber 1824.

The second fluid collection device 1812 further includes a second inlet 1826 in fluid communication with the first internal cavity 1804 of the first fluid collection device 1802. The second inlet 1826 is positioned between the first end 1814 of the second fluid collection device 1812 and the permeable membrane 1820. The second fluid collection device 1812 further includes a second outlet 1828 in fluid communication with the second inlet 1826 of the second fluid collection device 1812. The second outlet 1828 is positioned between the first end 1814 of the second fluid collection device 1812 and the permeable membrane 1820. Although Figure 18A illustrates the second inlet 1826 on a diagonally opposite side of the second fluid collection device 1812 from the second outlet 1828, in another embodiment the second inlet 1826 and the second outlet 1828 are positioned adjacent one another.

As such, the urine collection system 1800 provides a method for urine management when ab external suction line is not available (i.e., patient is in transit for X-ray or CT Scan) or suction line failure (i.e., malfunctioning vacuum pump, accidently turned off vacuum line, tube disconnected at the suction canister, vacuum leak at canister, or full canister may lead to lack of urine flow away from device). In patients with urinary incontinence, such lack of urine flow due to vacuum pump failure may lead to collection of urine on fragile skin areas, presenting incontinence-associated dermatitis (IAD) risk. To overcome such undesirable event, the urine collection system 1800 provides a safety feature to collect and "trap" urine away from the fragile skin only if such vacuum interruption occurs by collecting the urine within the device.

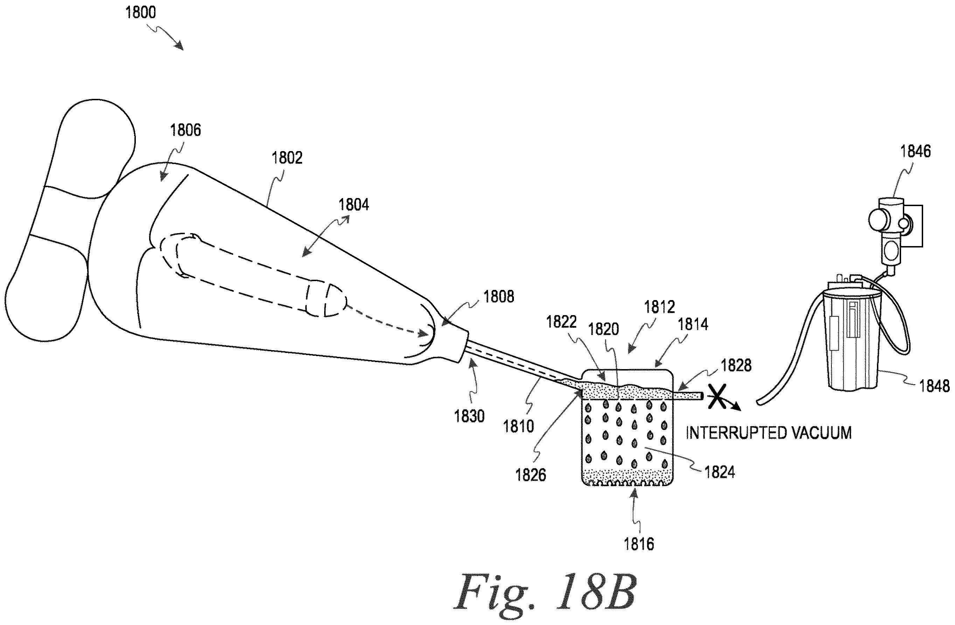

In particular, the second fluid collection device 1812 acts as a reservoir along a flow path between a first fluid collection device 1802 and a vacuum system. While suction is applied by the vacuum system, urine flows past the second fluid collection device 1812 to the vacuum system, as shown in Figure 18A. When no suction is applied, urine from the first fluid collection device 1802 is captured and retained by the second fluid collection device 1812, as shown in Figure 18B. In this arrangement, the urine collection system 1800 can mitigate leakage of urine when the first fluid collection device 1802 is disconnected from the vacuum system (either intentionally such as while moving a patient, or unintentionally such as in those situations stated above).

In one example, the second fluid collection device 1812 is removably coupled one of the first fluid collection device 1802 and the tube 1810. In one particular example, the first fluid collection device 1802 is connected to the assembly by means of male-female connector allowing "detachable" removal of the second fluid collection device 1812 in case significant fluid collection (e.g., patient voided a tiny amount during transport and clinician choses to just remove the second fluid collection device 1812 itself) without removing the complete urine collection system 1800.

In one example, as shown in Figures 18A-14B, the tube 1810 is positioned between the first fluid collection device 1802 and the second fluid collection device 1812. In such an example, a first end 1830 of the tube 1810 is in fluid communication with the first outlet 1808 of the first fluid collection device 1802, and a second end 1832 of the tube 1810 is in fluid communication with the second inlet 1826 of the second fluid collection device 1812.

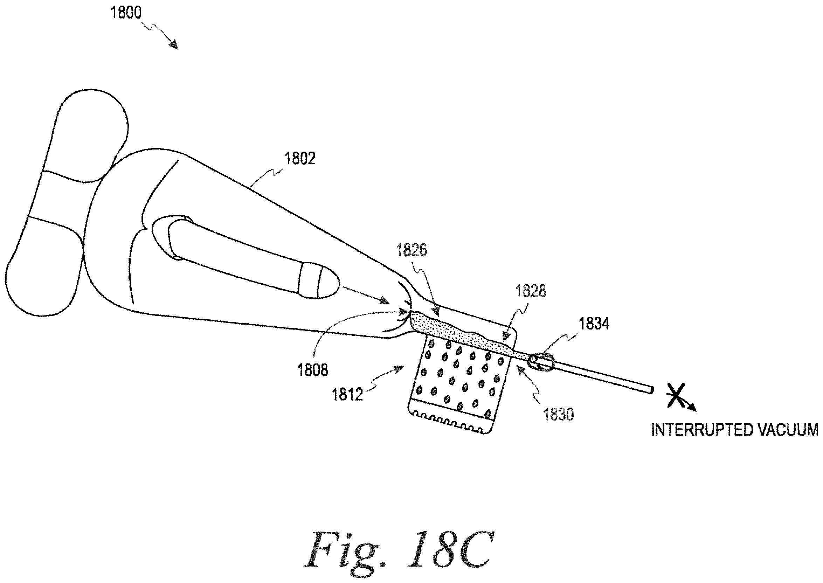

In another example, as shown in Figure 18C, the second fluid collection device 1812 is positioned between the first outlet 1808 of the first fluid collection device 1802 and the tube 1810. In such an example, the second inlet 1826 of the second fluid collection device 1812 is in fluid communication with the first outlet 1808 of the first fluid collection device 1802, and the second outlet 1828 of the second fluid collection device 1812 is in fluid communication with the first end 1830 of the tube 1810. In one such example, the urine collection system 1800 further includes a clamp 1834 positioned downstream from the second fluid collection device 1812 that when activated will prevent fluid flow through the tube 1810.

In various embodiments, the second fluid collection device 1812 comprises one or more of polyurethane, polyamide, polypropylene, polyethylene, polyvinyl chloride, ethylene vinyl acetate copolymer, polyether block amide polymers, silicone, or polyurethane thermoplastic. Further, a thickness of the second fluid collection device 1812 ranges from about 30 µm to about 5 mm.

The permeable membrane 1820 of the second fluid collection device 1812 may take a variety of forms. In one example, the permeable membrane 1820 comprises a polymer film. In another example, the permeable membrane 1820 includes one or more of a fenestration, one or more slits, or one or more slots. In another example, the permeable membrane comprises 1820 one or more drip filters. In another example, the permeable membrane 1820 includes a plurality of through-holes, and each of the plurality of through-holes have a length ranging from about 1 mm to about 5 mm. A collection rate of fluid through the permeable membrane 1820 ranges from about 1 mL/hour to about 400 mL/hour, more preferably from about 5 mL/hour to about 100 mL/hour in an absence of an external vacuum.

As shown in Figures 18A-18B, in one example the second fluid collection device 1812 is cylindrical in shape having a diameter ranging from about 30 mm to about 50 mm and a height ranging from about 100 mm to about 130 mm. In one example, the second chamber of the second fluid collection device has a volume ranging from about 100 mL to about 1,000 mL.

In order to accommodate such a range in volume, in one example as shown in Figure 18A, the second end 1816 of the second fluid collection device 1812 comprises an expandable material 1836 that is configured to expand unidirectionally with a weight of a fluid collected in the second chamber 1824. As such, the second chamber 1824 of the second fluid collection device 1812 has a first volume when a liquid is not present in the second chamber 1824, and a second volume that is greater than the first volume when liquid is present in the second chamber 1824. In one particular example, the expandable material 1836 comprises a polyurethane film. Other expandable materials are possible as well. In one such example, the first end 1814 of the second fluid collection device 1812 comprises a first material, and the second end 1816 of the second fluid collection device 1812 comprises a second material that is different than the first material. In one embodiment, to compensate for the weight of urine collected and associated downward pull, a tackier adhesive may be utilized in the first fluid collection device 1802 with peel strength can range from 0.1 to 10 N/cm, more preferably 0.25 to 3 N/cm. Alternatively, a thicker adhesive, or combination of adhesive may be utilized in some embodiment to improve the adherence of the first fluid collection device 1802 to the patient's skin.

In another example, as further shown in Figure 18A, an absorbent polymer 1838 is positioned at the second end 1816 of the second fluid collection device 1812 within the second chamber 1824. The absorbent polymer 1838 may be configured to convert any collected liquid into solid, thereby limiting the mobility of the liquid collected in the second chamber 1824. In such an embodiment, the absorbent polymer 1838 may comprise one or more of sodium polyacrylate, polyacrylate copolymers, cellulose, methacrylated chitosan, starch, or alginate. Further, in an embodiment including the absorbent polymer 1838, the urine collection system 1800 may further include a chemical dye, pigment, or a combination or colorant incorporated within the absorbent polymer 1838, where the chemical dye is only visible after absorption of urine into the absorbent polymer 1838. Such an embodiment may serve as a visual indicator for interrupted vacuum line to the patient caregiver indicating need for potential intervention.

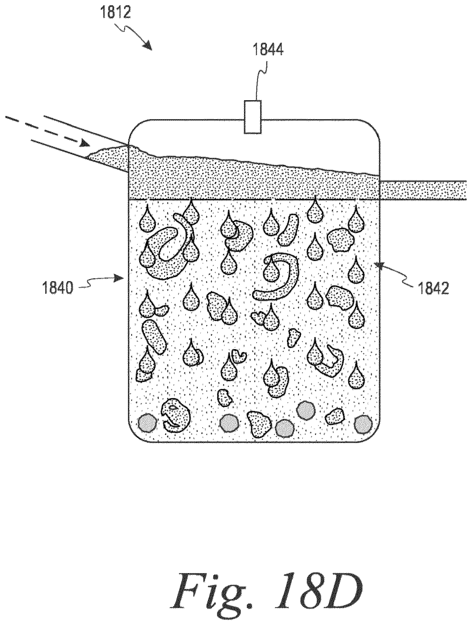

In one example, as shown in Figure 18D, the second fluid collection device 1812 comprises a polymer film 1840 positioned around an open cell foam 1842 to reduce the cost of the second fluid collection device 1812. Such an arrangement is configured to maintain the integrity of the second fluid collection device 1812 under a threshold magnitude of pressure (e.g., 40 mm of Hg) without significantly compromising the functionality of the second fluid collection device 1812.

In yet another example, as shown in Figure 18D, the urine collection system 1800 includes a vent hole 1844 positioned at the first end 1814 of the second fluid collection device 1812.

In another example, as shown in Figures 18A-18B, the urine collection system 1800 further includes a vacuum device 1846 in fluid communication with the second outlet 1828. The vacuum device 1846 is configured to apply a suction to the first fluid collection device 1802 to thereby remove urine from the first fluid collection device 1802. In such an arrangement, the urine collection system 1800 further includes a collection reservoir 1848 positioned between the second fluid collection device 1812 and the vacuum device 1846.

In use, as shown in Figure 18A, when suction is applied by the vacuum device 1846 above the threshold magnitude of pressure (e.g., greater than about 40 mm of Hg vacuum is applied by the vacuum device 1846), urine flows from the second inlet 1826, over the permeable membrane 1820, out the second outlet 1828, and to the collection reservoir 1848, as shown in Figure 18A. When suction is not applied or the suction is below the threshold magnitude of pressure (e.g., less than bout 40 mm of Hg vacuum is applied by the vacuum device 1846), urine passes from the first fluid collection device 1802 through the permeable membrane 1820 into the second chamber 1824 of the second fluid collection device 1812, and the urine is captured and retained by the second fluid collection device 1812, as shown in Figure 18B. As such, the urine collection system 1800 can mitigate leakage of urine when the first fluid collection device 1802 is disconnected from the vacuum system (either intentionally such as while moving a patient, or unintentionally such as in those situations stated above).

The first fluid collection device 1802 may take a variety of forms. As discussed above, in one example the first fluid collection device 1802 is configured to collect urine from a female anatomy. In another example, the first fluid collection device 1802 is configured to collect urine from a male anatomy. In such an example, the first fluid collection device 1802 may include any of the features of any of the urine collection devices 100, 200, 400, 600, 700, 800, 1100, 1400, 1500, 1600, 1700, 1900 described herein.

The description of the different advantageous arrangements has been presented for purposes of illustration and description, and is not intended to be exhaustive or limited to the embodiments in the form disclosed. Many modifications and variations will be apparent to those of ordinary skill in the art. Further, different advantageous embodiments may describe different advantages as compared to other advantageous embodiments. The embodiment or embodiments selected are chosen and described in order to explain the principles of the embodiments, the practical application, and to enable others of ordinary skill in the art to understand the disclosure for various embodiments with various modifications as are suited to the particular use contemplated.

The present disclosure also provides for the following Examples:
1. A urine collection system, comprising:
   a first fluid collection device including:
      a first internal cavity to collect urine discharged from a body of a user,
      a first inlet in fluid communication with the first internal cavity, and
      a first outlet in fluid communication with the first internal cavity;
   a tube in fluid communication with the first outlet; and
   a second fluid collection device having a first end and a second end opposite the first end, the second fluid collection device including:
      a second internal cavity defined between the first end and the second end of the second fluid collection device,
      a permeable membrane positioned within the second internal cavity between the first end and the second end of the second fluid collection device, thereby separating the second internal cavity into a first chamber and a second chamber,
      a second inlet in fluid communication with the first internal cavity of the first fluid collection device, wherein the second inlet is positioned between the first end of the second fluid collection device and the permeable membrane, and
      a second outlet in fluid communication with the second inlet of the second fluid collection device, wherein the second outlet is positioned between the first end of the second fluid collection device and the permeable membrane.
2. The urine collection system of example 1, wherein the tube is positioned between the first fluid collection device and the second fluid collection device, wherein a first end of the tube is in fluid communication with the first outlet of the first fluid collection device, and wherein a second end of the tube is in fluid communication with the second inlet of the second fluid collection device.
3. The urine collection system of example 1, wherein the second fluid collection device is positioned between the first outlet of the first fluid collection device and the tube, wherein the second inlet of the second fluid collection device is in fluid communication with the first outlet of the first fluid collection device, and wherein the second outlet of the second fluid collection device is in fluid communication with a first end of the tube.
4. The urine collection system of example 3, further comprising a clamp positioned downstream from the second fluid collection device that when activated will prevent fluid flow through the tube.
5. The urine collection system of example 1, wherein the second fluid collection device comprises one or more of polyurethane, polyamide, polypropylene, polyethylene, polyvinyl chloride, ethylene vinyl acetate copolymer, polyether block amide polymers, silicone, or polyurethane thermoplastic.
6. The urine collection system of example 1, wherein a thickness of the second fluid collection device ranges from about 30 µm to about 5 mm.
7. The urine collection system of example 1, wherein the permeable membrane comprises a polymer film.
8. The urine collection system of example 1, wherein the permeable membrane includes one or more of a fenestration, one or more slits, or one or more slots.
9. The urine collection system of example 1, wherein the permeable membrane comprises one or more drip filters.
10. The urine collection system of example 1, wherein the permeable membrane includes a plurality of through-holes, and wherein each of the plurality of through-holes have a length ranging from about 1 mm to about 5 mm.
11. The urine collection system of example 1, wherein a collection rate of fluid through the permeable membrane ranges from about 1 mL/hour to about 400 mL/hour in an absence of an external vacuum.
12. The urine collection system of example 1, wherein the second fluid collection device is cylindrical in shape having a diameter ranging from about 30 mm to about 50 mm and a height ranging from about 100 mm to about 130 mm.
13. The urine collection system of example 1, wherein the second chamber of the second fluid collection device has a volume ranging from about 100 mL to about 1,000 mL.
14. The urine collection system of example 1, wherein the second end of the second fluid collection device comprises an expandable material that is configured to expand unidirectionally with a weight of a fluid collected in the second chamber.
15. The urine collection system of example 1, wherein the first end of the second fluid collection device comprises a first material, and wherein the second end of the second fluid collection device comprises a second material that is different than the first material.
16. The urine collection system of example 1, further comprising:
   a vacuum device in fluid communication with the second outlet, wherein the vacuum device is configured to apply a suction to the first fluid collection device to thereby remove urine from the first fluid collection device.
17. The urine collection system of example 16, wherein the permeable membrane is configured to allow urine to pass therethrough only when less than about 40 mm of Hg vacuum is applied by the vacuum device.
18. The urine collection system of example 16, further comprising a collection reservoir positioned between the second fluid collection device and the vacuum device.
19. The urine collection system of example 1, further comprising an absorbent polymer positioned at the second end of the second collection device within the second chamber.
20. The urine collection system of example 19, wherein the absorbent polymer comprises one or more of sodium polyacrylate, polyacrylate copolymers, cellulose, methacrylated chitosan, starch, or alginate.
21. The urine collection system of example 19, further comprising a chemical dye incorporated within the absorbent polymer, wherein the chemical dye is only visible after absorption of urine into the absorbent polymer.
22. The urine collection system of example 1, wherein the second collection device includes a polymer film positioned around an open cell foam.
23. The urine collection system of example 1, wherein the second fluid collection device is removably coupled one of the first fluid collection device and the tube.
24. The urine collection system of example 1, further comprising a vent hole positioned at the first end of the second fluid collection device.
25. The urine collection system of example 1, further comprising:
   a collection member extending from a proximal end to a distal end, wherein the collection member has a longitudinal axis that extends between the proximal end and the distal end, wherein the proximal end comprises an opening that provides access to the first internal cavity, wherein the first internal cavity comprises a first chamber extending from the proximal end to the distal end, and wherein the first chamber is in fluid communication with a second chamber extending from the proximal end to the distal end.
26. The urine collection system of example 25, further comprising:
   a first attachment member extending from a bottom wall of the collection member at the proximal end, wherein the first attachment member defines an aperture; and
   a second attachment member extending from a top wall of the collection member at the proximal end.
27. The urine collection system of example 26, wherein the first attachment member comprises a first flexible member and a second flexible member that are movable relative to each other to facilitate access to the aperture in the first attachment member.
28. The urine collection system of example 27, wherein the first flexible member is separated from the second flexible member by a slit extending proximally from the aperture in the first attachment member.
29. The urine collection system of example 26, wherein the first attachment member comprises a first adhesive to assist in securing the first attachment member to the user, and wherein the second attachment member comprises a second adhesive to assist in securing the second attachment member to the user.
30. The urine collection system of example 29, wherein the first adhesive is coupled to an outer side of the first attachment member, and wherein the second adhesive is coupled to an inner side of the second attachment member.
31. The urine collection system of example 29, wherein the first adhesive extends entirely around the aperture in the first attachment member.
32. The urine collection system of example 26, wherein the first attachment member comprises a ring-shaped portion extending between an inner edge and an outer edge, wherein the inner edge defines a circumference of the aperture in the first attachment member, wherein the ring-shaped portion comprises an inner portion and an outer portion, wherein a first adhesive is coupled to the outer portion, and wherein the inner portion is suitable to deflect from the outer portion toward the collection member to expand a size of the aperture.
33. The urine collection system of example 25, further comprising an inner wall separating the first chamber and the second chamber, wherein the inner wall defines a plurality of perforations through which the first chamber fluidly communicates with the second chamber.
34. The urine collection system of example 33, further comprising:
   a spacer in the second chamber
   a permeable layer in the first chamber; and
   a wicking layer between the permeable layer and the inner wall,
   wherein the wicking layer is configured to move the urine from the first chamber toward the second chamber.
35. The urine collection system of example 34, wherein the opening in the collection member is defined by the inner wall and the top wall of the collection member.
36. The urine collection system of example 35, wherein the inner wall inhibits access to the second chamber from outside of the first chamber.
37. The urine collection system of example 1, wherein the first fluid collection device is configured to collect urine from a female anatomy.
38. The urine collection system of example 1, wherein the first fluid collection device is configured to collect urine from a male anatomy.
39. A urine collection device, comprising:
   an internal cavity to collect urine discharged from a body of a user;
   an outlet in fluid communication with the internal cavity; and
   a spacer positioned between the internal cavity and the outlet, wherein the spacer comprises a pliable film including a plurality of protrusions and a plurality of through-holes, and wherein the spacer is in fluid communication with the internal cavity and the outlet such that urine is directed from the internal cavity, through the spacer, and to the outlet.
40. The urine collection device of example 39, further comprising:
   a collection member extending from a proximal end to a distal end, wherein the collection member has a longitudinal axis that extends between the proximal end and the distal end, wherein the proximal end comprises an opening that provides access to the internal cavity, wherein the internal cavity comprises a first chamber extending from the proximal end to the distal end, and wherein the first chamber is in fluid communication with a second chamber extending from the proximal end to the distal end.
41. The urine collection device of example 40, further comprising:
   a first attachment member extending from a bottom wall of the collection member at the proximal end, wherein the first attachment member defines an aperture; and
   a second attachment member extending from a top wall of the collection member at the proximal end.
42. The urine collection device of example 41, wherein the first attachment member comprises a first flexible member and a second flexible member that are movable relative to each other to facilitate access to the aperture in the first attachment member.
43. The urine collection device of example 42, wherein the first flexible member is separated from the second flexible member by a slit extending proximally from the aperture in the first attachment member.
44. The urine collection device of example 41, wherein the first attachment member comprises a first adhesive to assist in securing the first attachment member to the user, and wherein the second attachment member comprises a second adhesive to assist in securing the second attachment member to the user.
45. The urine collection device of example 44, wherein the first adhesive is coupled to an outer side of the first attachment member, and wherein the second adhesive is coupled to an inner side of the second attachment member.
46. The urine collection device of example 44, wherein the first adhesive extends entirely around the aperture in the first attachment member.
47. The urine collection device of example 41, wherein the first attachment member comprises a ring-shaped portion extending between an inner edge and an outer edge, wherein the inner edge defines a circumference of the aperture in the first attachment member, wherein the ring-shaped portion comprises an inner portion and an outer portion, wherein a first adhesive is coupled to the outer portion, and wherein the inner portion is suitable to deflect from the outer portion toward the collection member to expand a size of the aperture.
48. The urine collection device of example 40, further comprising an inner wall separating the first chamber and the second chamber, wherein the inner wall defines a plurality of perforations through which the first chamber fluidly communicates with the second chamber.
49. The urine collection device of example 48, further comprising:
   a permeable layer in the first chamber; and
   a wicking layer between the permeable layer and the inner wall,
   wherein the wicking layer is configured to move the urine from the first chamber toward the second chamber.
50. The urine collection device of example 48, wherein the opening in the collection member is defined by the inner wall and the top wall of the collection member.
51. The urine collection device of example 50, wherein the inner wall inhibits access to the second chamber from outside of the first chamber.
52. The urine collection device of example 40, wherein the outlet comprises a port configured to couple to a drain tube of a vacuum device.
53. The urine collection device of example 53, wherein the port is rotatable with respect to the collection member.
54. The urine collection device of example 40, wherein the spacer extends distally from a distalmost point of the collection member.
55. The urine collection device of example 54, further comprising a first sheet of material coupled to a second sheet of material to thereby surround the spacer, and wherein the outlet is positioned the distalmost point of the spacer at a seam between the first sheet of material and the second sheet of material.
56. The urine collection device of example 39, wherein the spacer comprises one or more of polyurethane, polyamide, polypropylene, polyethylene, polyvinyl chloride, ethylene vinyl acetate copolymer, polyether block amide polymers, silicone, or polyurethane thermoplastic.
57. The urine collection device of example 39, wherein a thickness of the spacer ranges from about 30 µm to about 5 mm.
58. The urine collection device of example 39, wherein air is trapped in the plurality of protrusions of the spacer.
59. The urine collection device of example 39, wherein the plurality of protrusions in the spacer comprise one or more of circular, triangular, rectangular, or polygonal geometry.
60. The urine collection device of example 39, wherein a height of the plurality of protrusions range from about 500 µm to about 2 cm.
61. The urine collection device of example 60, wherein the height of the plurality of protrusions are uniform.
62. The urine collection device of example 60, wherein the height of the plurality of protrusions are non-uniform.
63. The urine collection device of example 39, wherein a lineal dimension of the plurality of protrusions range from about 1 mm to about 2 cm.
64. The urine collection device of example 39, wherein the plurality of protrusions are positioned on each side of the spacer.
65. The urine collection device of example 39, wherein the plurality of protrusions are encompassed between two flat film structures such that flow channels are present within a bilayer of the pliable film itself.
66. The urine collection device of example 39, wherein each of the plurality of through-holes have a length ranging from about 1 mm to about 5 mm.
67. The urine collection device of example 39, wherein the spacer is coated with one or more antimicrobial agents.
68. The urine collection device of example 67, wherein the one or more antimicrobial agents are selected from the group consisting of silver, acetic acid, polyhexamethylene biguanide, or combinations thereof.
69. The urine collection device of example 39, wherein the urine collection device is configured to collect urine from a female anatomy.
70. The urine collection device of example 39, wherein the urine collection device is configured to collect urine from a male anatomy.
71. A urine collection device comprising:
   a collection member extending from a proximal end to a distal end, wherein the collection member defines an internal cavity to collect urine discharged from a body of a user; and
   an outlet in fluid communication with the internal cavity, wherein the outlet comprises:
      a port support structure at the distal end of the collection member, and
      a port extending from the port support structure, wherein the port is configured to couple to a drain tube.
72. The urine collection device of example 71, wherein the port support structure is in the internal cavity of the collection member.
73. The urine collection device of any one of examples 71-72, wherein the port support structure extends across a width of the collection member.
74. The urine collection device of any one of examples 71-73, wherein the port support structure is formed from a material having a rigidity that is greater than a rigidity of a material of the collection member.
75. The urine collection device of any one of examples 71-74, further comprising a spacer in the internal cavity of the collection member,
   wherein the port support structure defines a recess at a proximal side of the port structure, and
   wherein the spacer is received in the recess of the port support structure.
76. A urine collection device comprising:
   a collection member extending from a proximal end to a distal end, wherein the proximal end comprises an opening that provides access to an internal cavity of the collection member, wherein the internal cavity comprises a first chamber in fluid communication with a second chamber, wherein the collection member comprises a plurality of seams on opposing sides of the opening at the proximal end;
   a spacer in the second chamber;
   a first attachment member extending from a bottom wall of the collection member at the proximal end, wherein the first attachment member defines an aperture;
   a second attachment member extending from a top wall of the collection member at the proximal end; and
   an outlet for egressing urine from the internal cavity of the collection member,
   wherein the collection member is suitable to (i) direct urine from the first chamber to the second chamber and (ii) direct the urine in the second chamber distally toward the outlet.
77. The urine collection device of example 76, wherein each of the plurality of seams extend inwardly from a respective lateral edge of the collection member towards a middle portion of the collection member, and wherein the opening is at the middle portion between the plurality of seams.
78. The urine collection device of any one of examples 76-77, wherein the collection member comprises a first sheet of material coupled to a second sheet of material, and wherein the first sheet of material is coupled to the second sheet of material at the plurality of seams.
79. A urine collection device comprising:
   a collection member extending from a proximal end to a distal end, wherein the proximal end comprises an opening that provides access to an internal cavity of the collection member, wherein the internal cavity comprises a first chamber in fluid communication with a second chamber;
   a spacer in the second chamber;
   a first attachment member extending from a bottom wall of the collection member at the proximal end, wherein the first attachment member defines an aperture;
   a second attachment member extending from a top wall of the collection member at the proximal end; and
   an outlet for egressing urine from the internal cavity of the collection member,
   wherein the collection member is suitable to (i) direct urine from the first chamber to the second chamber and (ii) direct the urine in the second chamber distally toward the outlet,
   wherein the collection member comprises a plurality of tabs on opposing sides of the opening at the proximal end,
   wherein each tab is movable relative to the collection member, and
   wherein each tab is configured to couple to an outer surface of the collection member to cover at a respective portion of the opening at the proximal end.
80. The urine collection device of example 79, wherein the collection member comprises a first sheet of material coupled to a second sheet of material, wherein the plurality of tabs extend from the first sheet of material at the proximal end, and
   wherein each tab is configured to extend over the portion of the opening and couple to the second sheet of material.
81. The urine collection device of any one of examples 79-80, wherein each tab comprises an adhesive that is configured to couple the tab to the outer surface of the collection member.
82. The urine collection device of any one of examples 39-81, further comprising a reinforcement member on at least one of a top wall or a bottom wall of the collection member, wherein the reinforcement member comprises an additional layer of material formed from the same material as the at least one of the top wall or the bottom wall are formed, wherein the reinforcement member is at a distal portion of the collection member.

## Claims

1. A urine collection device comprising:
a collection member extending from a proximal end to a distal end, wherein the proximal end comprises an opening that provides access to an internal cavity of the collection member, wherein the internal cavity comprises a first chamber in fluid communication with a second chamber, wherein the collection member comprises a plurality of seams on opposing sides of the opening at the proximal end;
a spacer in the second chamber;
a first attachment member extending from a bottom wall of the collection member at the proximal end, wherein the first attachment member defines an aperture;
a second attachment member extending from a top wall of the collection member at the proximal end; and
an outlet for egressing urine from the internal cavity of the collection member,
wherein the collection member is suitable to (i) direct urine from the first chamber to the second chamber and (ii) direct the urine in the second chamber distally toward the outlet.

2. The urine collection device of claim 1, wherein each of the plurality of seams extend inwardly from a respective lateral edge of the collection member towards a middle portion of the collection member, and wherein the opening is at the middle portion between the plurality of seams.

3. The urine collection device of claim 1 or 2, wherein the collection member comprises a first sheet of material coupled to a second sheet of material, and wherein the first sheet of material is coupled to the second sheet of material at the plurality of seams.

4. The urine collection device of any one of claims 1 to 3, wherein the first attachment member is integrally formed with the top wall and the second attachment member is integrally formed with the bottom wall.

5. The urine collection device of claims 3 and 4, wherein the first sheet of material provides the top wall of the collection member and the first attachment member, and wherein the second sheet of material provides the bottom wall and the second attachment member.

6. The urine collection device of any one of claims 1 to 5, wherein the first attachment member comprises a first flexible member and a second flexible member that are movable relative to each other.

7. The urine collection device of claim 6, wherein the first flexible member is separated from the second flexible member by a slit.

8. The urine collection device of any one of claims 1 to 7, wherein the first attachment member comprises a first adhesive to assist in securing the first attachment member to the user, and wherein the second attachment member comprises a second adhesive to assist in securing the second attachment member to the user.

9. The urine collection device of claim 8, wherein the first adhesive extends entirely around the aperture in the first attachment member.

10. The urine collection device of any one of claims 1 to 5, wherein the outlet comprises a port rotatably coupled to the collection member.

11. The urine collection device of claim 10, wherein the port comprises a joint that is configured to allow the port to rotate about an axis relative to the collection member.

12. The urine collection device of claim 10 or 11, wherein the port is freely rotatable by more than 360 degrees relative to the collection member.

13. The urine collection device of claim 10 or 11, wherein the port is rotatable by less than 360 degrees relative to the collection member.

14. The urine collection device of any one of claims 10 to 13, wherein the port is rotatable about an axis relative to the collection member.

15. The urine collection device of any one of claims 1 to 14, wherein the first attachment member includes an outer side for contacting a pelvic area of a user and an inner side facing the collection member when the urine collection device is secured to the user, wherein the aperture extends through the first attachment member from the outer side to the inner side, and wherein the device is configured such that the user's penis extends through the aperture and through the opening of the collection member when the urine collection device is secured to the user.
